(19) Europäisches Patentamt
European Patent Office
Office européen des brevets

(11) Publication number: **0 147 765 A2**

(12) **EUROPEAN PATENT APPLICATION**

(21) Application number: 84115660.7

(22) Date of filing: 17.12.84

(51) Int. Cl.[4]: G 03 C 7/32
//C07D257/04, C07D249/18

(30) Priority: 15.12.83 JP 237101/83

(43) Date of publication of application: 10.07.85 Bulletin 85/28

(84) Designated Contracting States: DE GB

(71) Applicant: FUJI PHOTO FILM CO., LTD.
210 Nakanuma Minami Ashigara-shi
Kanagawa 250-01(JP)

(72) Inventor: Kobayashi, Hidetoshi c/o Fuji Photo Film Co., Ltd.
No. 210, Nakanuma, Minami
Ashigara-shi, Kanagawa(JP)

(72) Inventor: Mihayashi, Keiji c/o Fuji Photo Film Co., Ltd.
No. 210, Nakanuma, Minami
Ashigara-shi, Kanagawa(JP)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22(DE)

(54) **Silver halide photographic light-sensitive material.**

(57) A silver halide photographic light-sensitive material comprising a support and at least one silver halide emulsion layer wherein the silver halide photographic light-sensitive material contains at least one compound represented by the following general formula (I):

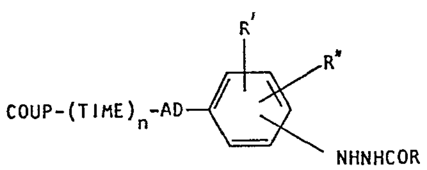

wherein COUP represents a coupler residue capable of undergoing a coupling reaction with an oxidation product of an aromatic primary amine developing agent; TIME represents a timing group which is released from COUP the coupling reaction and subsequently releases

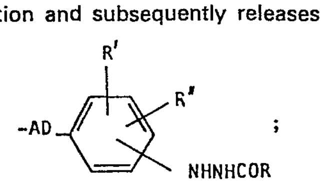

;

n is 0 or 1;

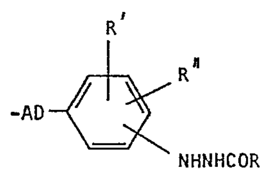

represents a group capable of being released from COUP by the coupling reaction when n is 0, or a group capable of being released from TIME after TIME which has been released from COUP when n is 1, AD being adsorptive to silver halide particles; R represents an alkyl group, an aryl group or a heterocyclic group; R' and R", which may be the same or different, each represents a hydrogen atom or a substituent; and –NHNHCOR may be directly connected to AD when AD is combined with the benzene ring to form a condensed ring system.

The silver halide photographic light-sensitive material containing the compound represented by general formula (I) is improved in stability during storage and has a high sensitivity.

EP 0 147 765 A2

Croydon Printing Company Ltd.

# SILVER HALIDE PHOTOGRAPHIC LIGHT-SENSITIVE MATERIAL

## FIELD OF THE INVENTION

The present invention relates to a silver halide photographic light-sensitive material and more particularly, to a silver halide photographic light-sensitive material which has excellent stability during storage as well as high sensitivity.

## BACKGROUND OF THE INVENTION

It is well known that color images can be obtained by exposing a silver halide photographic light-sensitive material to light, followed by color development during which a reaction between an oxidized aromatic primary amine developing agent and a dye forming coupler takes place. The subtractive color process is generally used for color reproduction, in which cyan, magenta and yellow color images, which are complementary to red, green and blue colors, respectively, are formed. The reaction between the coupler and the oxidation product of a color developing agent proceeds at an active point of the coupler. A coupler having a hydrogen atom at its active point (i.e., 4-equivalent coupler) stoichiometrically requires as an oxidizing agent 4 moles of silver halide having a development center for forming 1 mole of dye through the coupling reaction. A coupler having at its active point a group capable of being released in the form

of an anion requires only 2 moles of silver halides having a development center for forming 1 mole of dye (i.e., a 2-equivalent coupler). Accordingly, by using a 2-equivalent coupler, the amount of silver halide to be used in a light-sensitive layer can be reduced and the layer can be made thinner, so that the time required for processing such a light-sensitive material can be shortened and the color images obtained can have an improved sharpness, compared with a light-sensitive material in which a 4-equivalent coupler is used. In addition, the coupling activity of a 2-equivalent coupler with a color developing agent can be widely varied, depending on the property of the releasable group used.

A 2-equivalent coupler capable of releasing a group having a development-inhibiting effect is known as a development inhibitor releasing coupler (or DIR coupler). Such couplers are capable of inhibiting development in proportion to the quantity of developed silver and, therefore, can be effective for improving graininess, controlling gradation and improving color reproducibility of the image. Couplers of this type can also be used in diffusion transfer processes, exerting their effects upon adjacent layers.

A 2-equivalent coupler can also be provided with a releasable group containing a diffusible dye portion. This class of couplers, which are referred to as

diffusible dye-releasing couplers, can be utilized in a diffusion transfer process in which a dye image is formed from diffused dyes in an image receiving layer.

A certain colored 2-equivalent coupler, which is referred to as a colored coupler, can also be used to provide the masking effect necessary for color correction of dye images.

As illustrated above, 2-equivalent couplers can have various functions, depending on the releasable groups contained therein.

Recent silver halide photographic light-sensitive materials, and in particular, those used for photography, require both increased sensitivity, as is illustrated by ISO 1000 films, and an improvement in image quality due to the minituarization of film sizes. To increase sensitivity, investigations have been made on a variety of techniques, including, e.g., large size silver halide grains, couplers with higher activities and acceleration of development. However, the increase in sensitivity obtainable using large size silver halide grains seems to be reaching its limit, as reported by G.C. Farnell and J.B. Chanter in J. Photogr. Sci., Vol. 9, page 75 (1961). Accordingly, this technique is not expected to make much contribution in the future. In addition, the use of large size silver halide grains is accompanied by various disadvantages, such as deterioration in

graininess. Couplers having higher activities have also been studied extensively. Such couplers, however, have not significantly increased sensitivities of silver halide photographic light-sensitive materials, and have proved to be disadvantageous as to graininess. With regard to acceleration of development, various development accelerators, including hydrazine compounds, have been used in a silver halide emulsion layer or a developing solution, mainly with black-and-white photographic light-sensitive materials. However, in most cases, the incorporation of development accelerators into an emulsion layer or a developing solution is accompanied by disadvantages such as increased fog and deterioration in graininess, and are for this reason impractical.

Couplers which imagewise release development accelerators or fogging agents have also been proposed. For example, couplers releasing thiocyanic acid ions which accelerate the solution physical development are disclosed in U.S. Patents 3,214,377 and 3,253,924, Japanese Patent Application (OPI) No. 17437/76, etc. Further, couplers releasing acyl hydrazines are described in Japanese Patent Application (OPI) No. 150845/82, couplers releasing hydroquinone or aminophenol developing agents are described in Japanese Patent Application (OPI) No. 138636/82 and couplers releasing fogging agents such as thiocarbonyl compounds, are described in Japanese Patent

Application No. 161515/82.

However, most of these compounds have low stability and they undesirably affect several photographic properties, for example, by increaseing fog, densitization, etc., and by decreasing their sensitizing function over time when they are incorporated into silver halide photographic light-sensitive materials. Further, these compounds have the disadvantage that color mixing occurs, that color reproducibility is degraded and that the graininess increases since the releasable groups diffuse into other layers to cause development acceleration or to form fog due to their large diffusibility, when they are incorporated into a particular light-sensitive layer which is sensitive to light of a specific color.

## SUMMARY OF THE INVENTION

It is, therefore, an object of the present invention to provide a silver halide photographic light-sensitive material having both high sensitivity and excellent stability during storage.

Another object of the present invention is to provide a silver halide photographic light-sensitive material which can form color images having good color reproducibility.

Other objects of the present invention will be apparent from the following detailed description and

examples.

These objects of the present invention can be achieved by a silver halide photographic light-sensitive material comprising a support and at least one silver halide emulsion layer wherein the silver halide photographic light-sensitive material contains at least one compound represented by the following general formula (I):

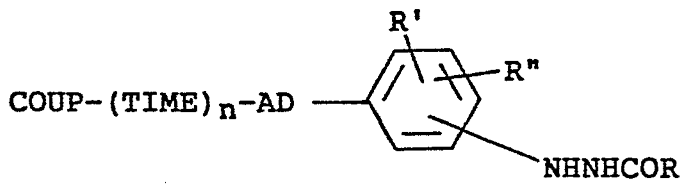

(I)

wherein COUP represents a coupler residue capable of undergoing a coupling reaction with an oxidation product of an aromatic primary amine developing agent; TIME represents a timing group which is released from COUP by the coupling reaction and subsequently releases

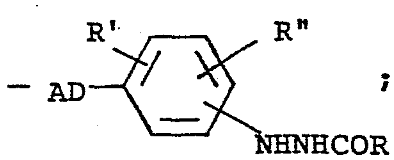

; n is 0 or 1;

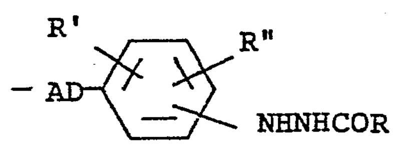

represents a group capable of being released from COUP by the coupling reaction when n is 0 or a group capable of being released from TIME after TIME has been released from COUP when n is 1; AD being adsorptive to silver halide particles; R represents an alkyl group,

an aryl group or a heterocyclic group; R' and R", which may be the same or different, each represents a hydrogen atom or a substituent described below; provided that NHNHCOR may be directly connected to AD when AD is combined with the benzene ring to form a condensed ring system.

DETAILED DESCRIPTION OF THE INVENTION

The compounds represented by the general formula (I) which can be used in the present invention are described in detail in the following.

The coupler residue represented by COUP includes a residue derived from a cyan, magenta, yellow or non-color-forming coupler. Examples of coupler residues represented by COUP include residues of cyan couplers, such as phenol couplers and naphthol couplers, etc.; residues of magenta couplers, such as 5-pyrazolone couplers, pyrazolobenzimidazole couplers, pyrazolotriazole couplers, cyanoacetylcoumarone couplers, open chain acylacetonitrile couplers and indazolone couplers, etc.; residues of yellow couplers, such as benzoylacetanilide couplers, pivaloylacetanilide couplers and malondianilide couplers, etc.; and residues of non-color-forming couplers, such as open chain or cyclic active methylene compounds (e.g., indanones, cyclopentanones, diesters of malonic acid, imidazolinones, oxazolidines, thiazolinones, etc.).

Of the coupler residues represented by COUP, those which are preferably used in the present invention include the residues represented by the following general formula (II), (III), (IV), (V), (VI), (VII), (VIII), (IX) or (X):

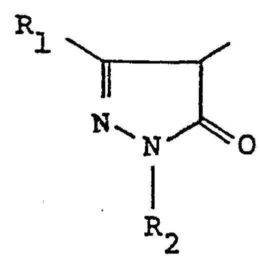

(II)

wherein $R_1$ represents an acylamido group, an anilino group or a ureido group; and $R_2$ represents a phenyl group which may be substituted with one or more substituents selected from a halogen atom, an alkyl group, an alkoxy group or a cyano group.

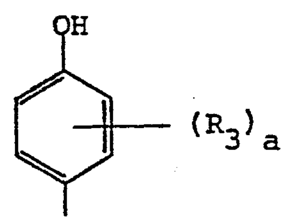

(III)

wherein $R_3$ represents a halogen atom, an acylamido group or an aliphatic group; a is an integer of 1 to 4, and when a is at least 2, $R_3$ may be the same or different.

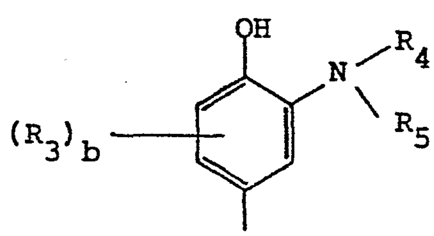

(IV)

wherein $R_3$ has the same meaning as defined above; $R_4$ and $R_5$, which may be the same or different, each represents an aliphatic group, an aromatic group, a carbamoyl group or a heterocyclic group, provided that not more than one of $R_4$ and $R_5$ may be a hydrogen atom; and b is 0 or an integer of 1 to 3, provided when b is at least 2, $R_3$ may be the same or different.

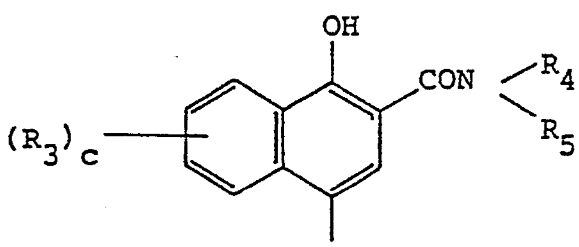

(V)

wherein $R_3$, $R_4$ and $R_5$ each has the same meaning as defined above; and c is 0 or an integer of 1 to 5, provided that when c is at least 2, $R_3$ may be the same or different.

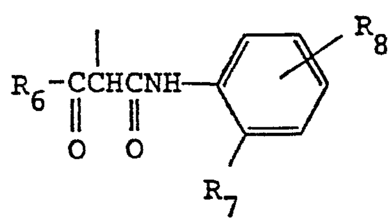

(VI)

wherein $R_6$ represents a tertiary alkyl group or an aromatic group; $R_7$ represents a hydrogen atom, a halogen atom or an alkoxy group; and $R_8$ represents an acylamido group, an aliphatic group, an alkoxycarbonyl group, a sulfamoyl group, a carbamoyl group, an alkoxy group, a halogen atom or a sulonamido group.

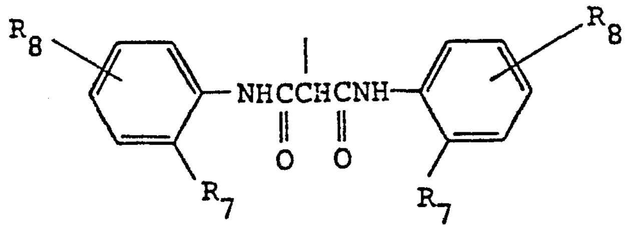

(VII)

wherein $R_7$ and $R_8$ each has the same meaning as defined above.

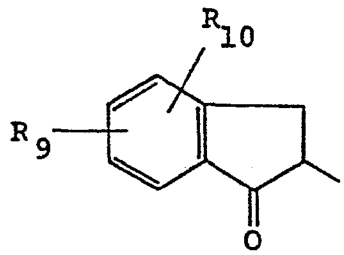

(VIII)

wherein $R_9$ represents an aliphatic group, an alkoxy group, a mercapto group, an alkylthio group, an acylamido group, an alkoxycarbonyl group, a sulfonamido group, a carbamoyl group, a sulfamoyl group, an alkoxysulfonyl group, an aryloxysulfonyl group, an acyl group, a diacylamino group, an alkylsulfonyl group or an arylsulfonyl group; and $R_{10}$ represents a hydrogen atom, a halogen atom, an alkoxy group, an acyl group, a nitro group, an alkylsulfonyl group or an arylsulfonyl group; or the group represented by general formula (VIII) above may be in the form of an enol ester thereof.

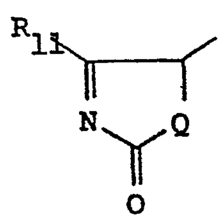

(IX)

wherein $R_{11}$ represents an aliphatic group or an aromatic

group; and Q represents an oxygen atom, a sulfur atom or a nitrogen atom.

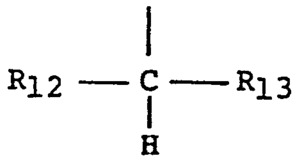

(X)

wherein $R_{12}$ and $R_{13}$, which may be the same or different, each represents a group selected from the group consisting of

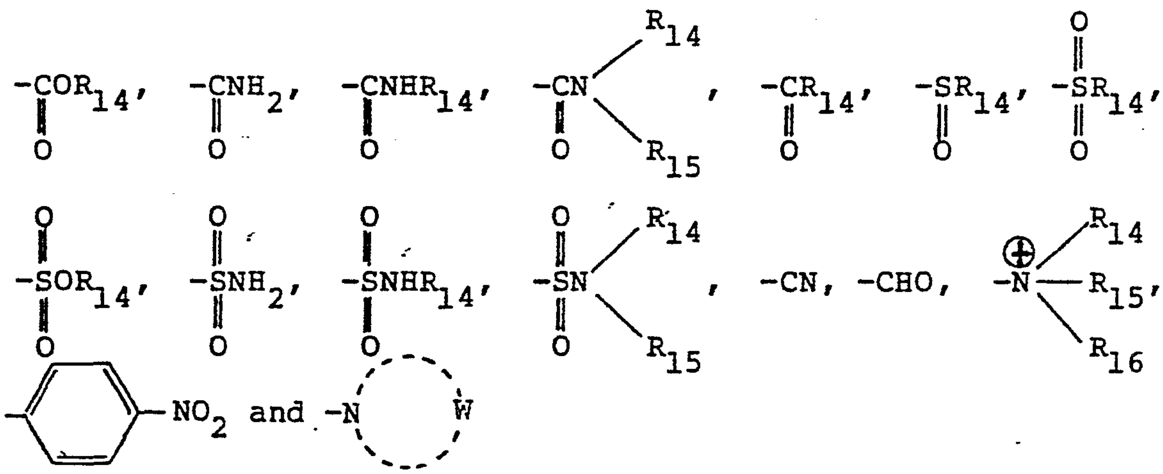

wherein $R_{14}$, $R_{15}$ and $R_{16}$, which may be the same or different, each represents a hydrogen atom, an aliphatic group, an aromatic group or a heterocyclic group; and W represents a non-metallic atomic group necessary to form a 5-membered or 6-membered ring together with the nitrogen atom; and $R_{12}$ and $R_{13}$ may combine to form a 5-membered or 6-membered ring together with a non-metallic atomic group necessary for completing the ring.

Examples of the group having a timing function, as represented by TIME in the general formula (I) include

a group which is released from COUP by the coupling reaction and then undergoes an intramolecualr displacement reaction (e.g., the timing groups described in U.S. Patent 2,428,962 and Japanese Patent Application (OPI) No. 56837/82), a group wich is released from COUP by the coupling reaction and then undergoes an electron transfer reaction via a conjugated system (e.g., the timing groups described in British Patent 2,072,363A and Japanese Patent Application (OPI) Nos. 154234/82 and 188035/82), and a group which by the coupling reaction with the oxidation product with an aromatic primary amine developing agent, generates a site which can be subjected to further coupling reaction and undergoes a second coupling reaction whereby it has a timing function (e.g., the timing group described in Japanese Patent Application (OPI) No. 111536/82). The TIME may include a group in multiple stages combining these mechanisms.

Examples of the groups capable of adsorbing to silver halide grains represented by AD include a group derived from a nitrogen-containing heterocyclic ring having a dissocialbe hydrogen atom (for example, pyrrole, imidazole, pyrazole, triazole, tetrazole, benzimidazole, benzopyrazole, benzotriazole, uracil, tetraazaindene, imidazotetrazole, pyrazolotriazole, pentaazaindene, etc.), a group derived from a heterocyclic ring having a mercapto group (for example, 2-mercaptobenzothiazole, 2-mercaptopyrimidine, 2-mercaptobenzoxazole, 1-phenyl-5-

mercaptotetrazole, etc.), etc.

When AD has a benzene ring condensed with another benzene ring or a heterocyclic ring, the condensed benzene ring may also play a part of the benzene ring to which a group of -NHNHCOR is connected.

AD may be connected to 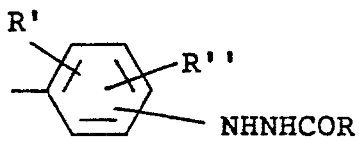

by a divalent linking group, for example, an alkylene group, an alkenylene group, an arylene group, -O-, -S-, CONH-, $-SO_2NH-$, -COO-, -SO-, $-SO_2-$, -NHCONH- or -NHCSNH-.

AD may be connected to TIME or COUP at the adsorbing site to silver halide particles (for example, a nitrogen atom of benzotriazole or a sulfur atom of 1-phenyl-5-mercaptotetrazole) but the invention is not limited to this embodiment. If AD is not connected to TIME or COUP at the adsorbing site, it is preferred that a hydrogen atom is connected to the adsorbing site or that the adsorbing site is blocked with a group that is hydrolyzable in a developing solution (for example, an acetyl group, a benzoyl group or a methanesulfonyl group,) or a group that is releasable in a developing solution (for example, a 2-cyanoethyl group or a 2-methanesulfonylethyl group).

R preferably represents an alkyl group having from 1 to 11 carbon atoms, an alkenyl group having from

2 to 11 carbon atoms, an aryl group having from 6 to 12 carbon atoms or a heterocyclic group having from 1 to 12 carbon atoms. The alkyl group or the alkenyl group represented by R may be straight chain or branched chain and may be further substituted with, for example, a halogen atom, a hydroxy group, an alkoxy group, an aryloxy group, an alkylthio group, an arylthio group, an acyl group, an alkoxycarbonyl group, a carbamoyl group, an alkylsulfonyl group, an arylsulfonyl group, a sulfamoyl group, a carbonamido group, a sulfonamido group, a carboxy group, a sulfo group, a heterocyclic group, a cyano group, an imido group or an amino group, although the substituent is not limited thereto. The heterocyclic group represented by R is preferably a 5-membered to 7-membered heterocyclic group containing a nitrogen atom, a sulfur atom or an oxygen atom (for example, a 2-pyridyl group, a 4-pyridyl group, a 2-furyl group, a 2-thenyl group, a pyranyl group, an imidazolyl group or an imidazolinyl group). The aryl group or the heterocyclic group represented by R may be condensed with a benzene ring or a hetero ring and may be substituted with any of the substituents described for R above.)

Specific examples of R are set forth below, but the present invention is not to be construed as limited thereto:

$CH_3$-, $C_2H_5$-, $C_3H_7$-, (i)-$C_3H_7$-, (t)-$C_4H_9$-, $C_{11}H_{23}$-,

$CH_3OCH_2CH_2$-, $HOCH_2$-, $ClCH_2$-, $Cl_2CH$-, $CF_3$-, 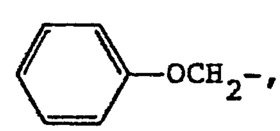

$CH_2=CH-$, $CH_2=CH-CH_2-$, 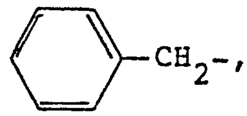

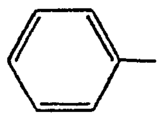 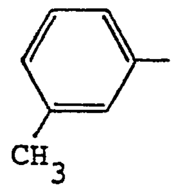 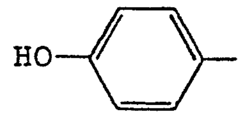

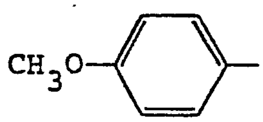 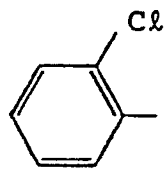 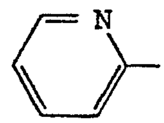

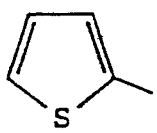 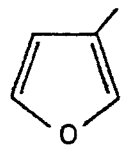 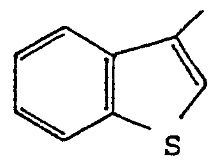

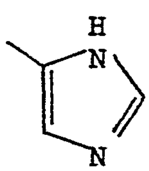 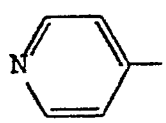 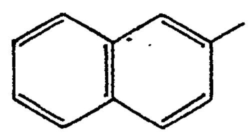

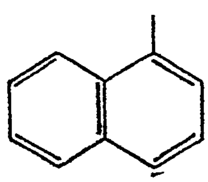 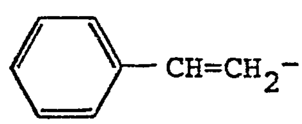

R' and R" each represents a hydrogen atom, an alkyl group, an alkoxy group or a halogen atom.

Specific examples of AD are set forth below, although the present invention is not limited thereto:

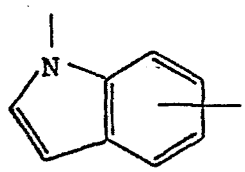

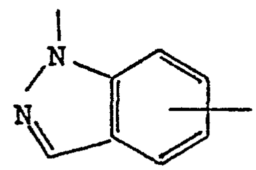

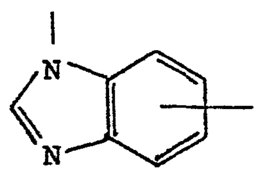

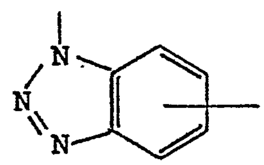

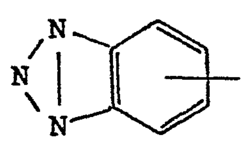

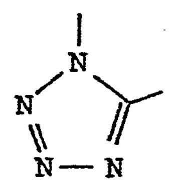

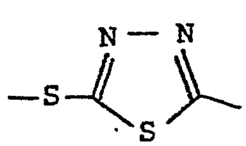

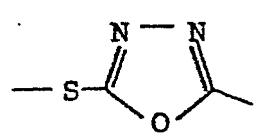

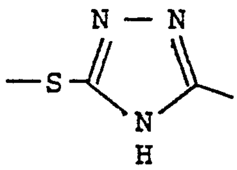

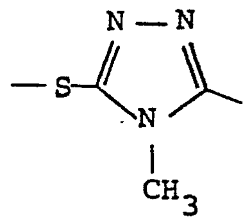

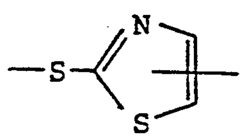

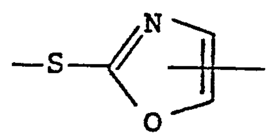

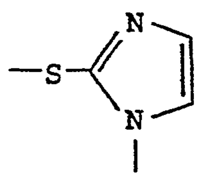

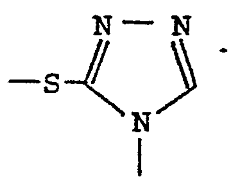

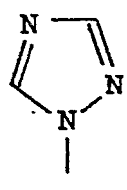

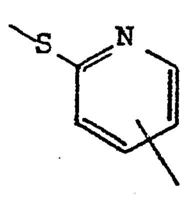

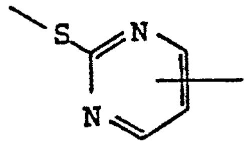

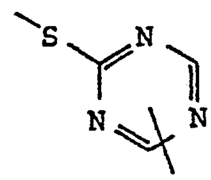

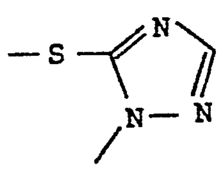

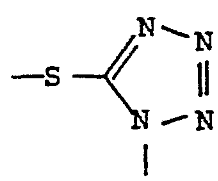

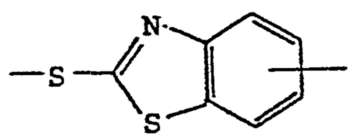

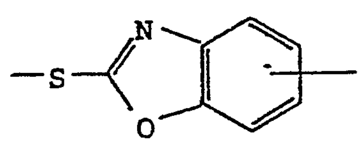

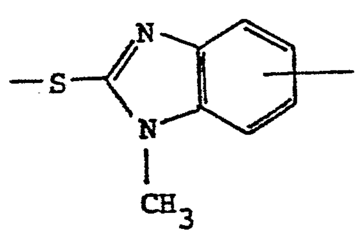

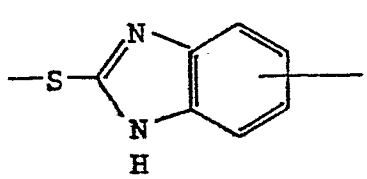

Specific example of 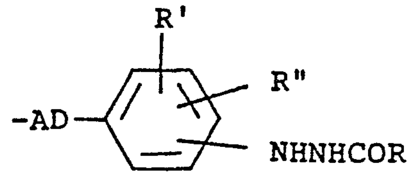

are set forthe below, although the present invention is not limited thereto.

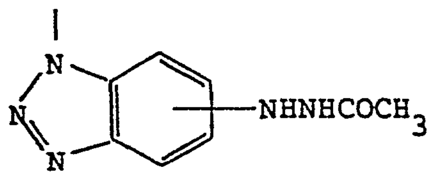

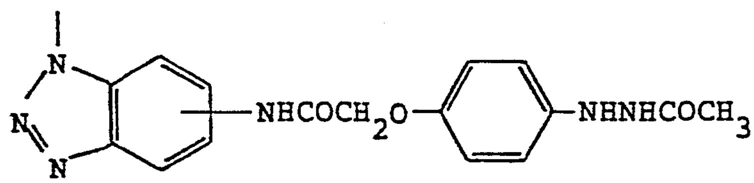

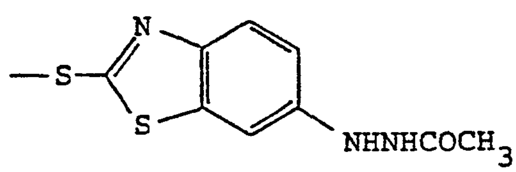

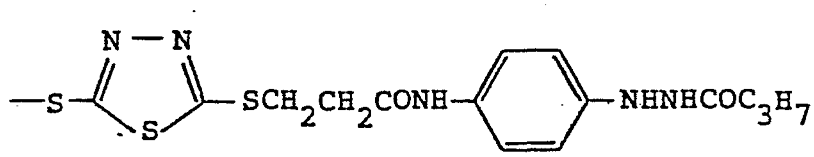

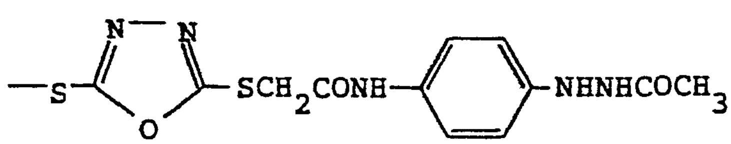

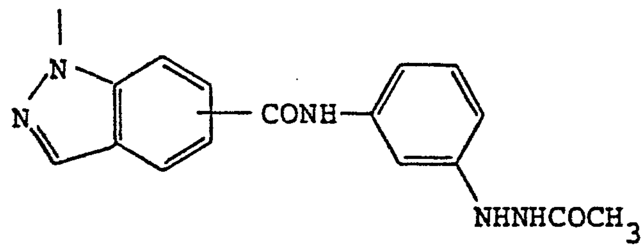

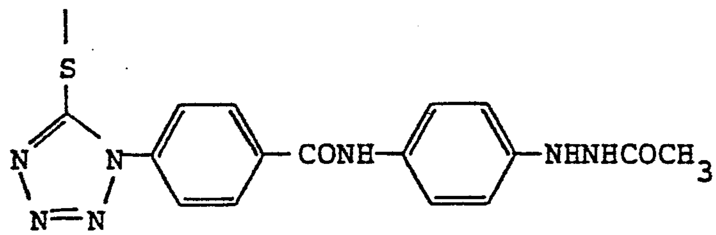

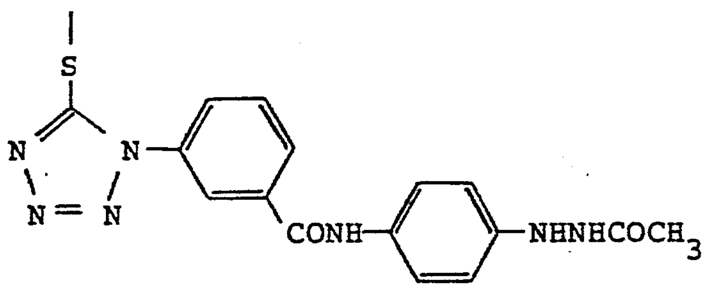

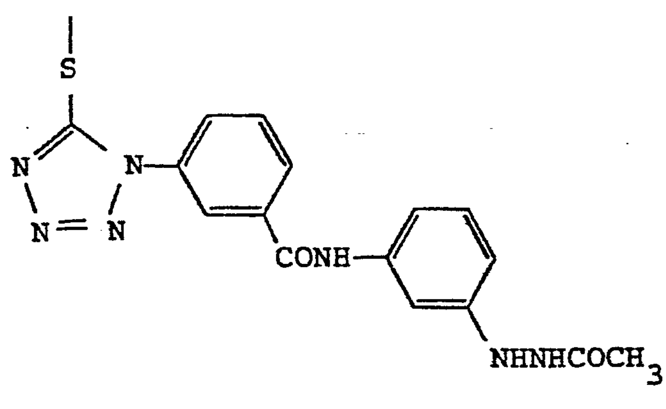

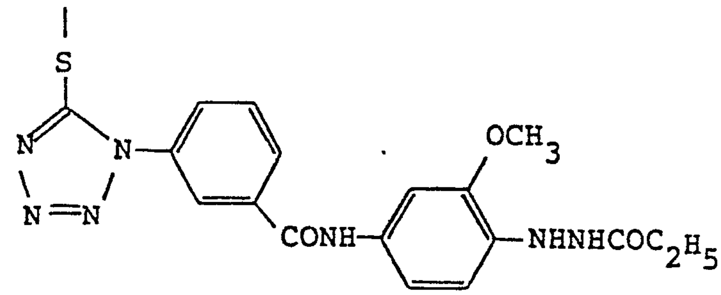

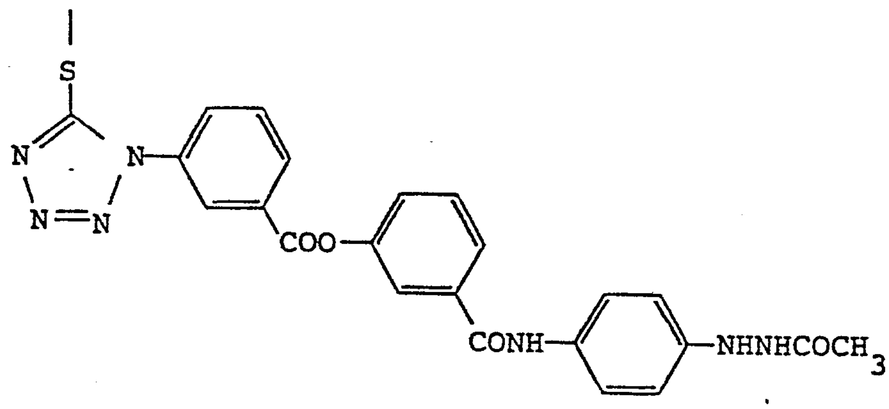

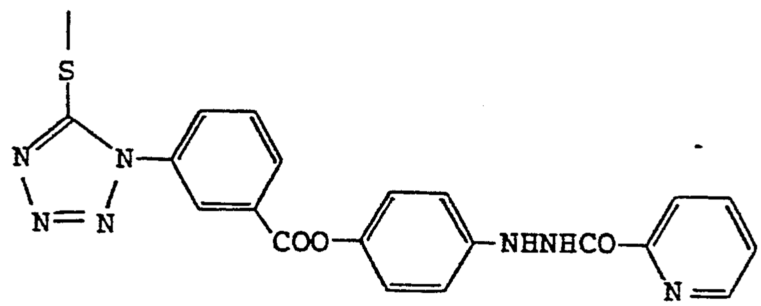

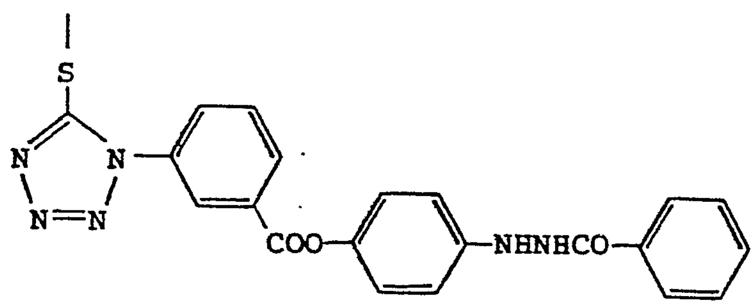

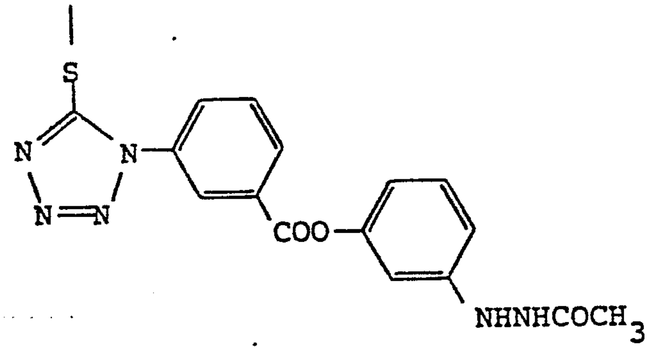

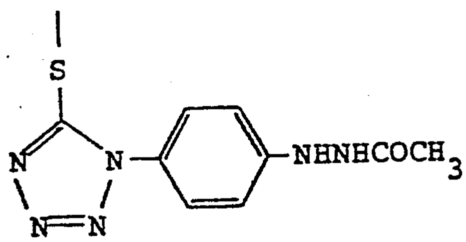

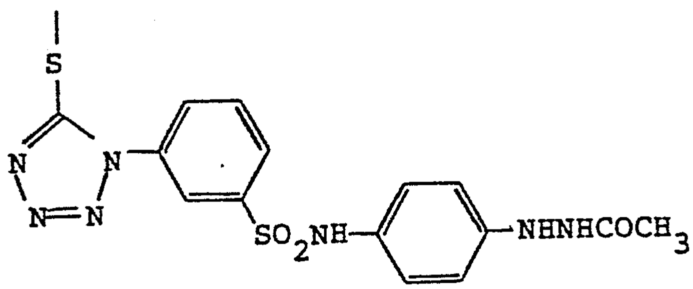

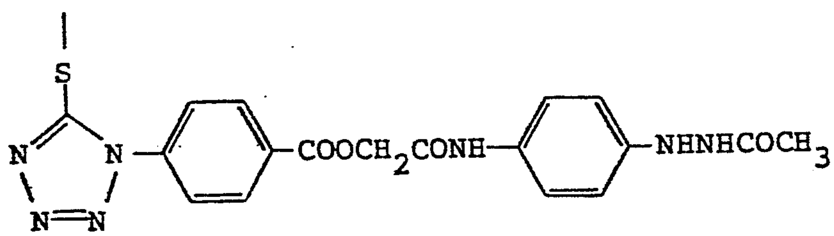

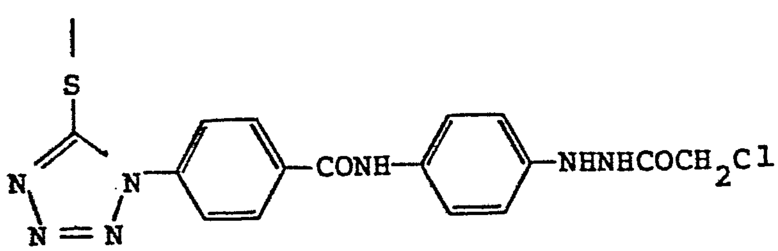

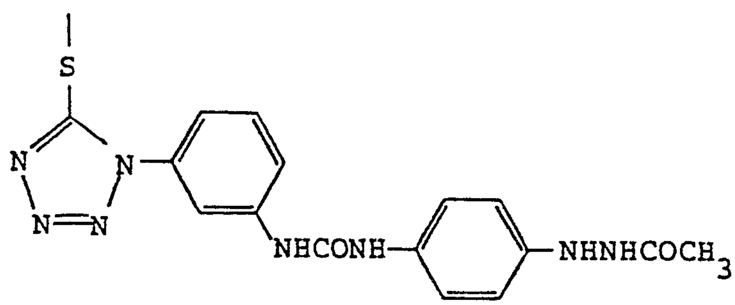

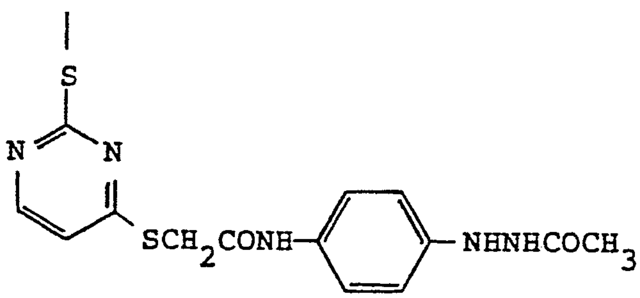

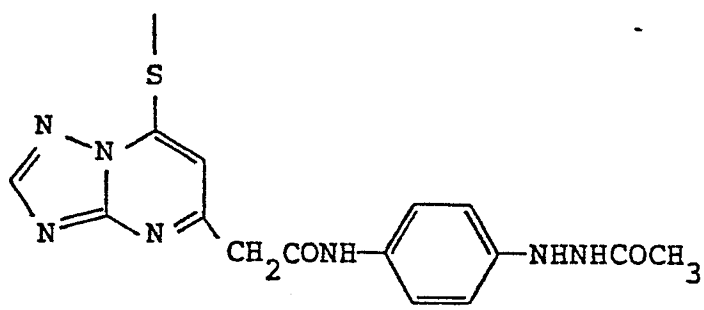

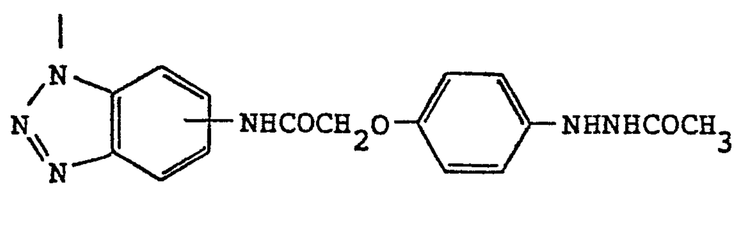

Specific examples of the compounds used in the present invention are set forth below, but the present invention should not be construed as being limited thereto.

1.

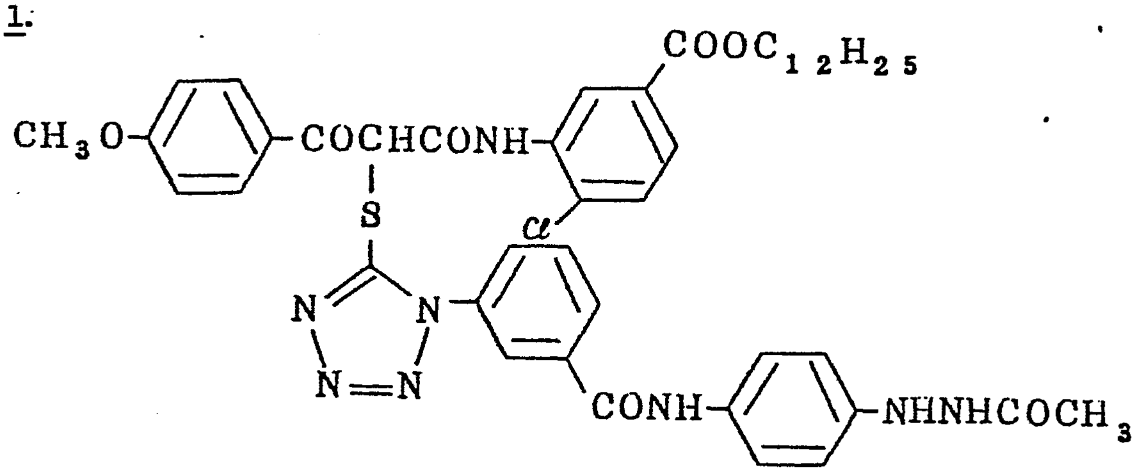

2.

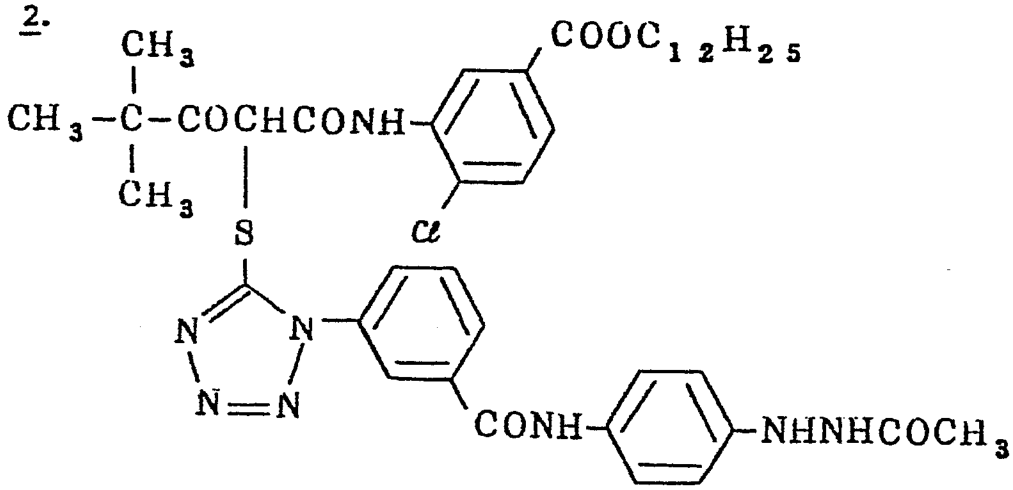

3

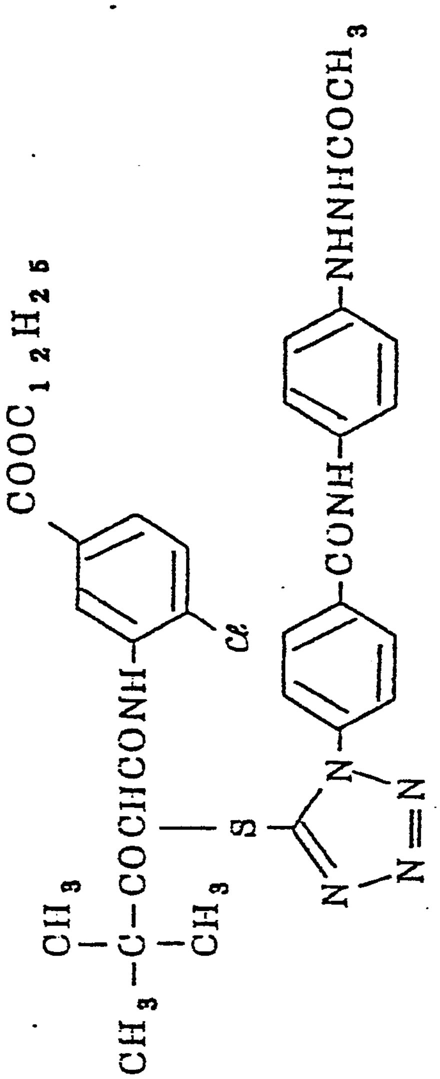

4

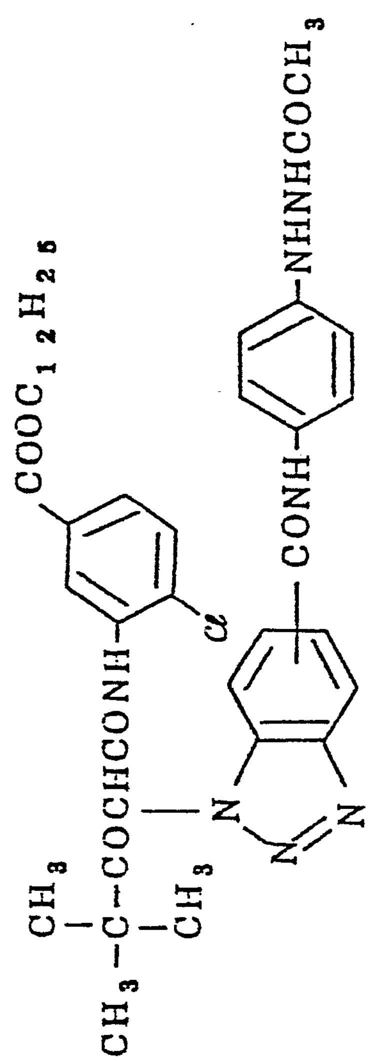

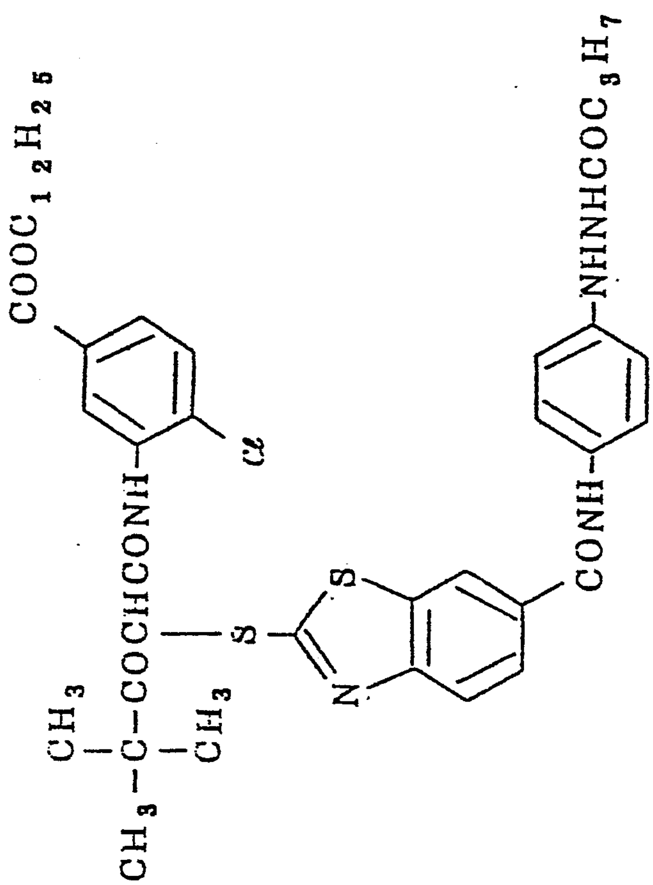

6.

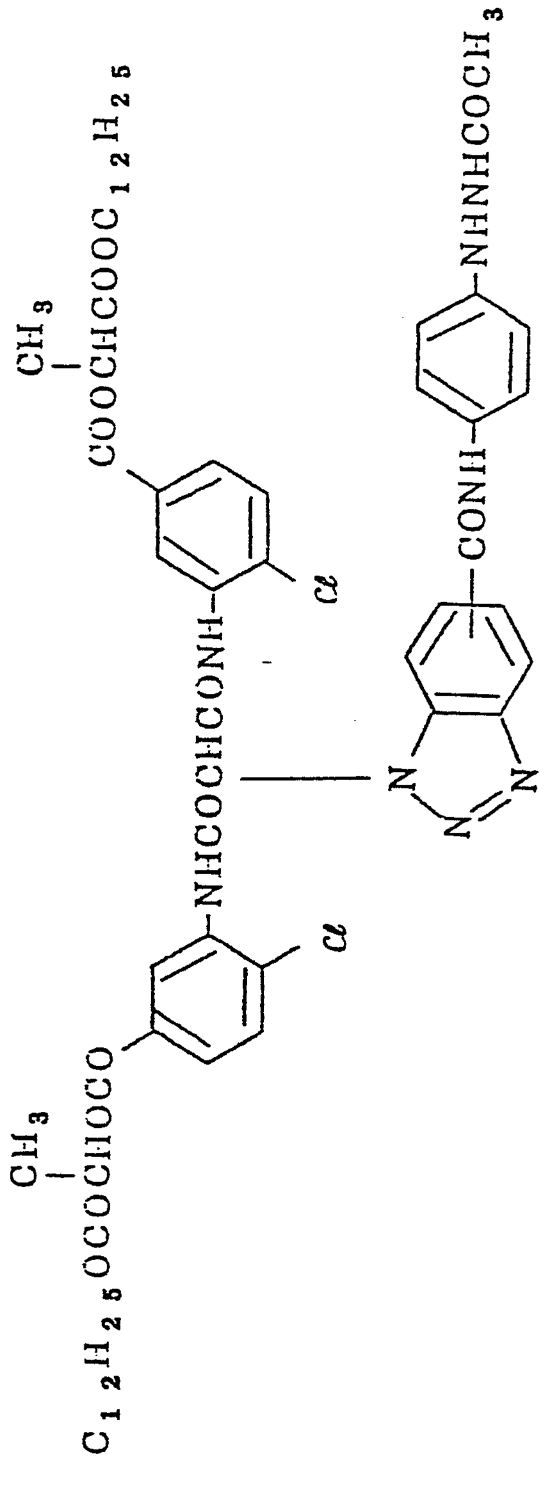

7

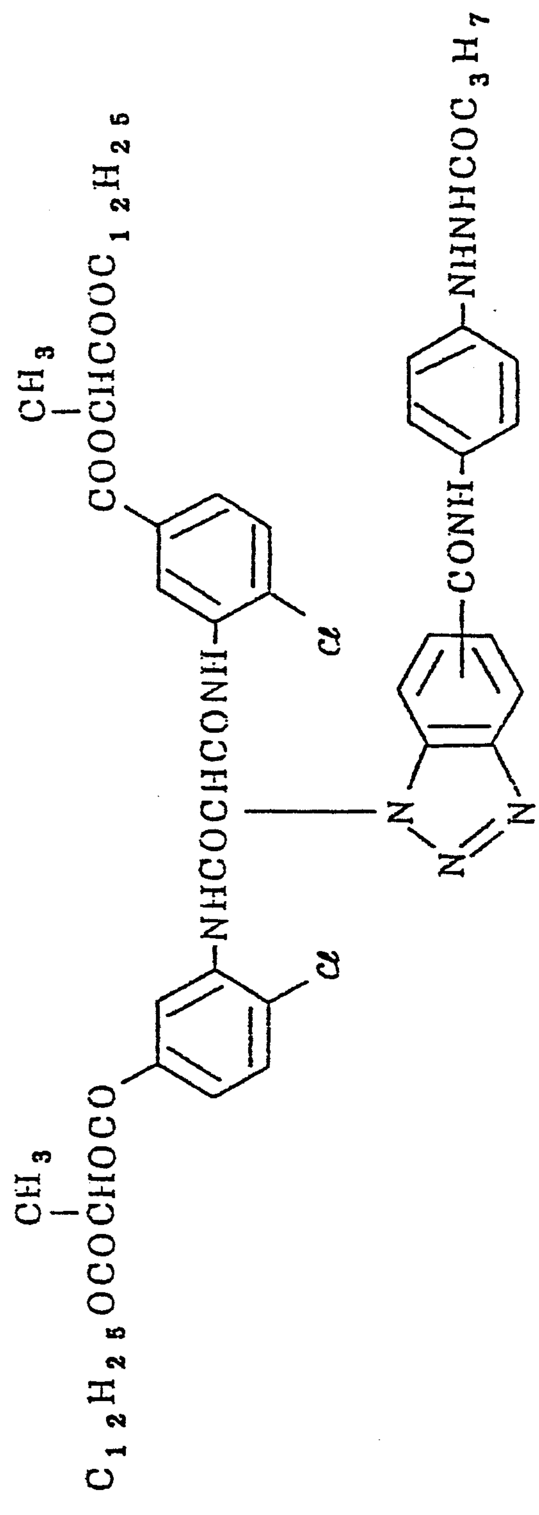

8.

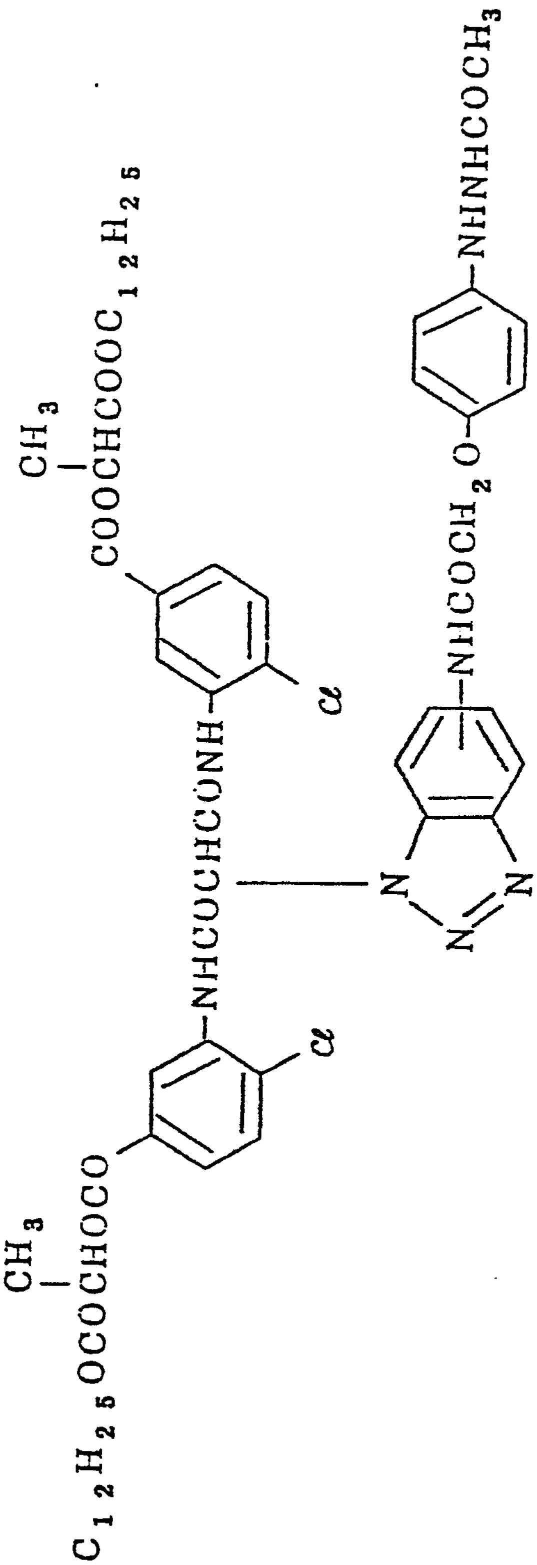

9.

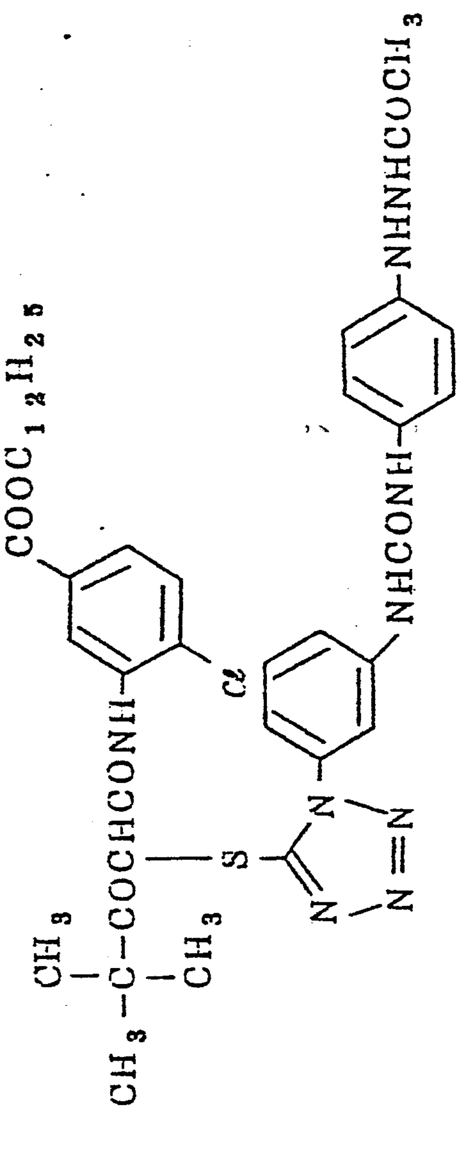

10.

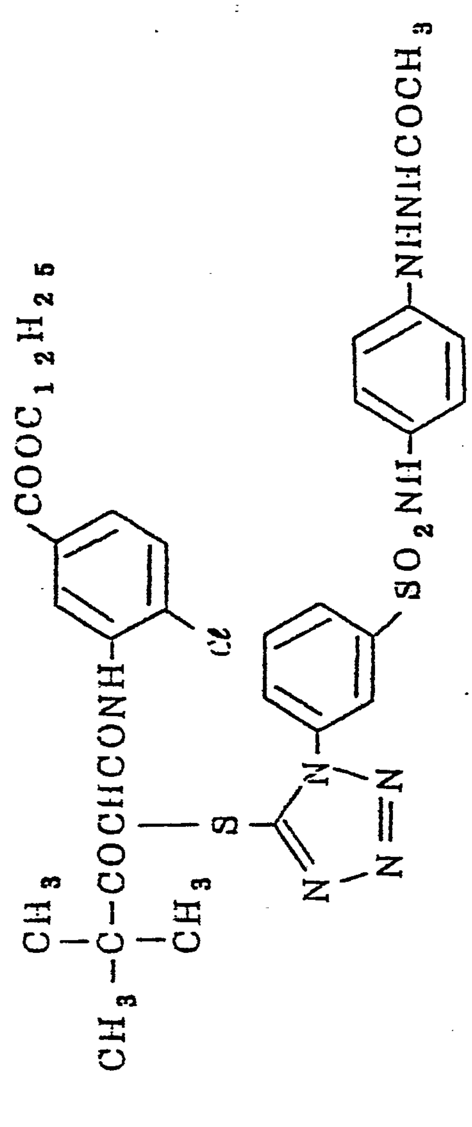

11.

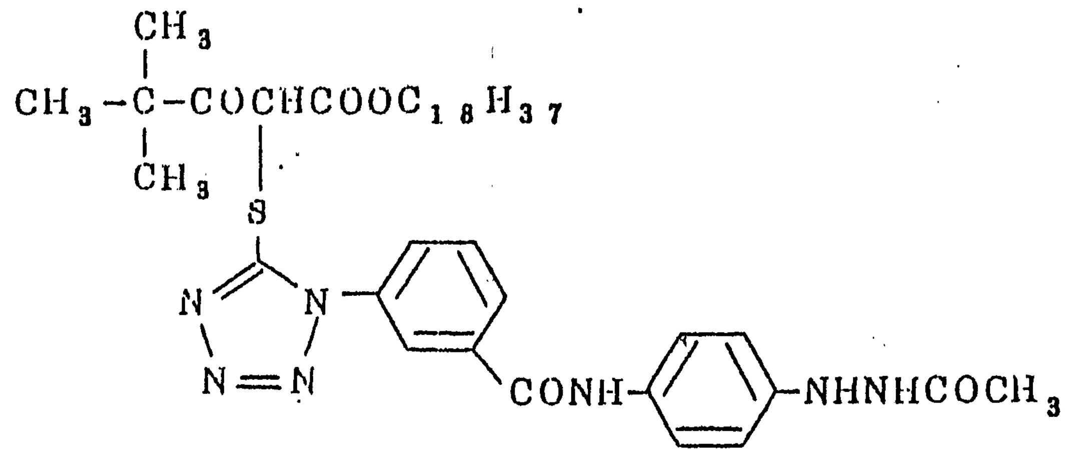

12.

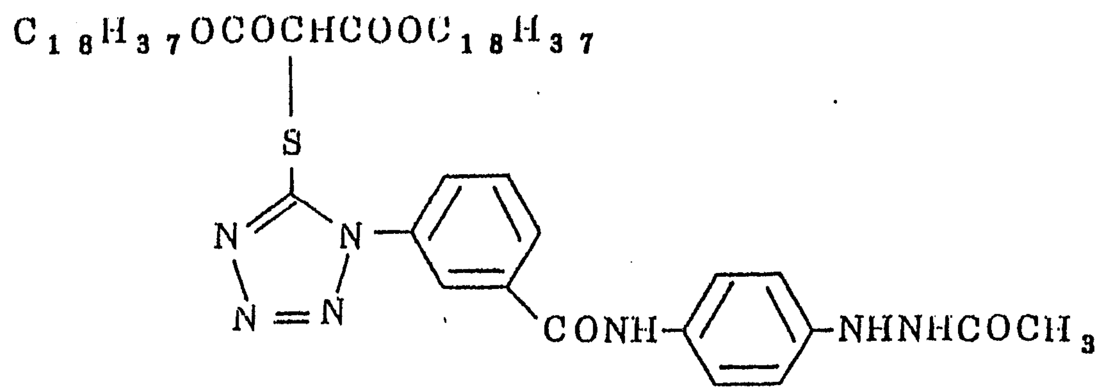

13.

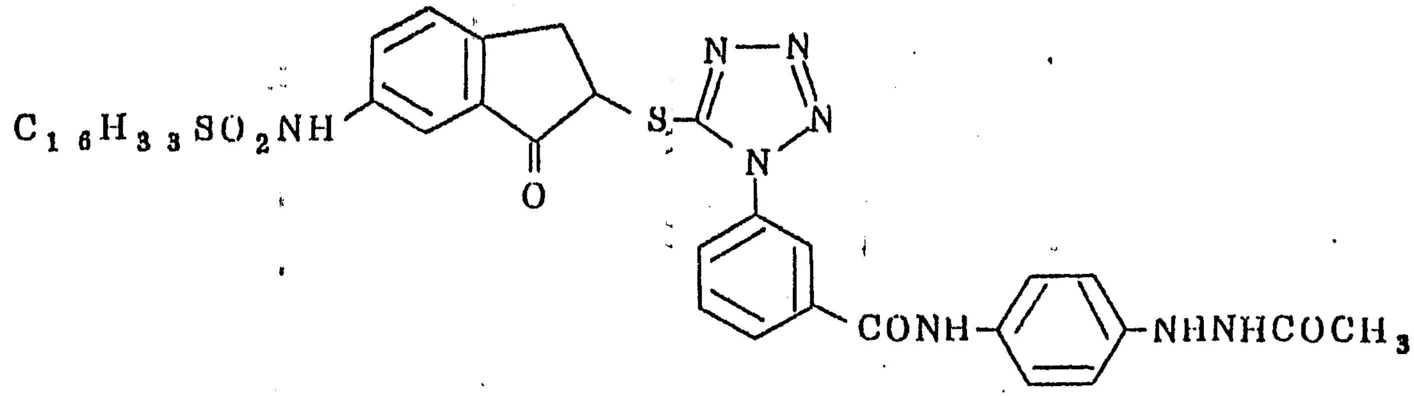

14.

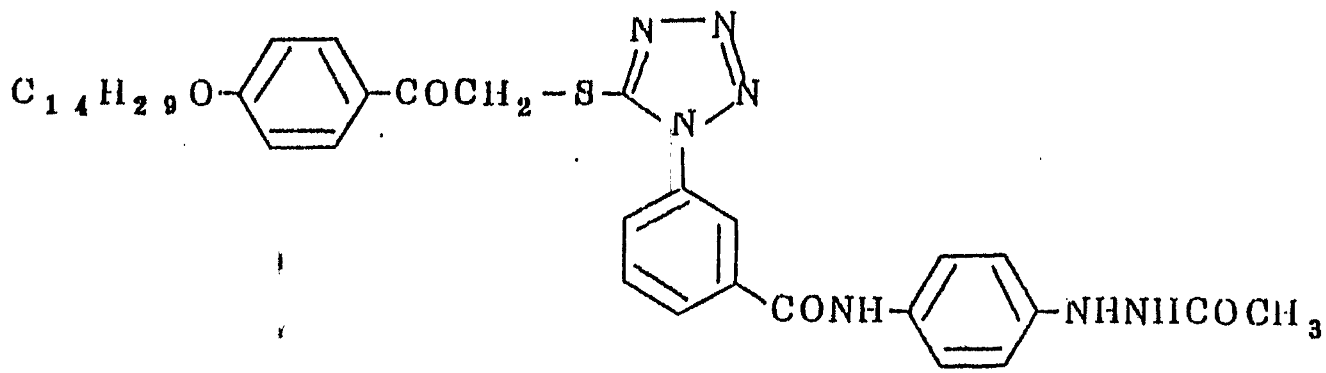

15.

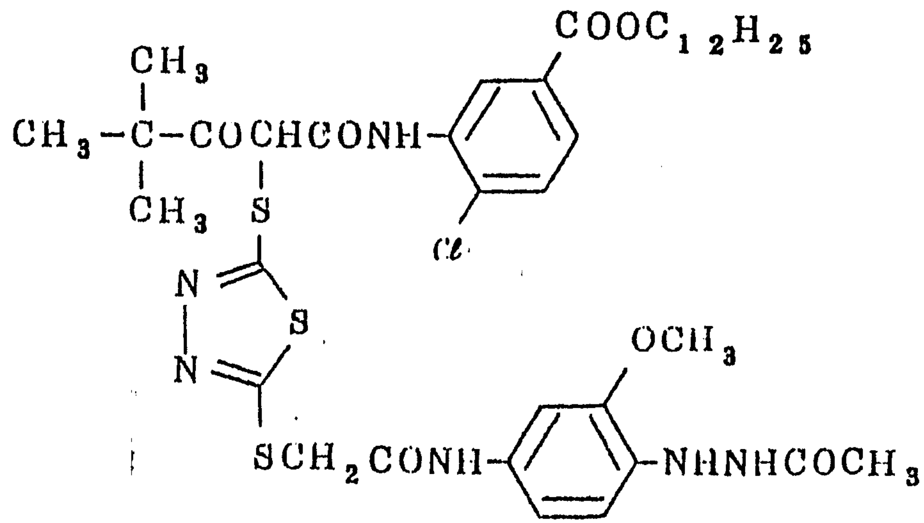

16.

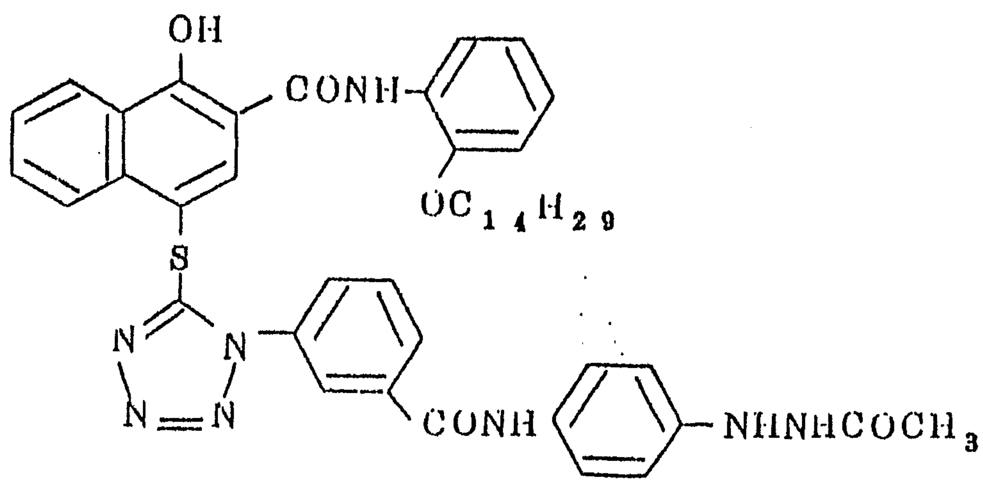

17

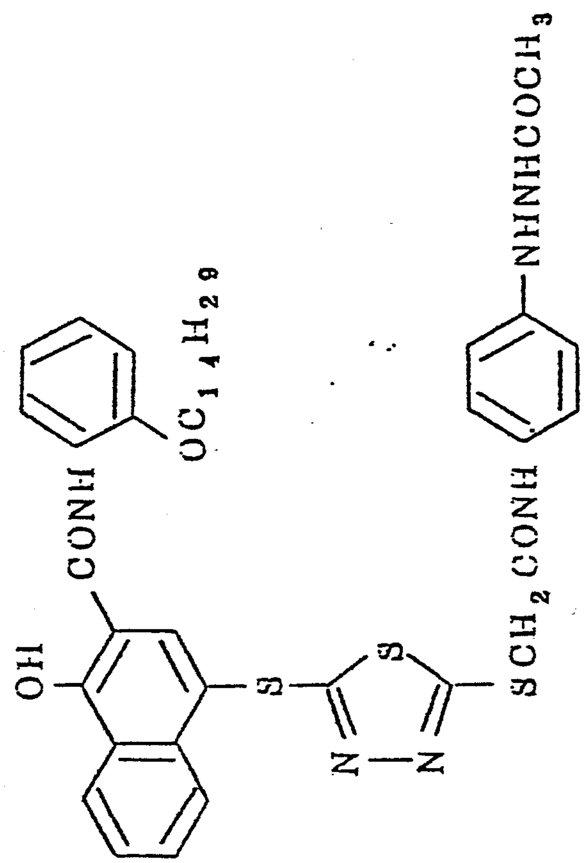

The compounds according to the present invention can be synthesized, for example, using the reaction as described in Japanese Patent Application (OPI) No. 150845/82. Examples of synthesis are set forth below.

SYNTHESIS EXAMPLE 1

Synthesis of Compound 1

Synthesis of ethyl m-isothiocyanatobenzoate

82.5 g of ethyl m-aminobenzoate was dissolved in 500 ml of toluene and to the solution was added dropwise 83.4 g of N,N-diethylthiocarbamoyl chloride at room temperature with stirring over a period of about 1 hour. The solution was then refluxed by heating for 5 hours. The toluene was distilled off under reduced pressure, and to the residue was added 300 ml of ethyl acetate, and the ethyl acetate solution was washed with water. The ethyl acetate was distilled off under reduced pressure and the residue was further subjected to vacuum distillation to obtain 80 g of ethyl m-isothiocyanatobenzoate as an oily product. Boiling Point: 125°C/0.6 mmHg. Yield: 77%.

Synthesis of 1-(3-carboxyphenyl)-5-mercaptotetrazole

500 ml of water was added to a mixture of 69 g of ethyl m-isothiocyanatobenzoate and 26 g of sodium azide and the mixture was refluxed by heating for 2 hours. After removing the insoluble material precipitated by filtration, concentrated hydrochloric acid was added to the filtrate whereby the filtrate was acidified (pH:

about 2). The crystals of 1-(3-ethoxycarbonylphenyl)-5-mercaptotetrazole thus precipitated were collected by filtration, to which were added 30 g of sodium hydroxide and 300 ml of water and the mixture was heated to 70°C and stirred for 30 minutes. The reaction solution was neutralized with concentrated hydrochloric acid and the crystals thus precipitated were collected by filtration. By recrystallization of the crude crystals from methanol, 32 g of 1-(3-carboxyphenyl)-5-mercaptotetrazole was obtained. Yield: 42%. Melting Point: 181 to 182°C.

Synthesis of α-(4-methoxybenzoyl)-α-[1-(3-carboxyphenyl)-5-tetrazolylthio]-2-chloro-5-dodecyloxycarbonylacetanilide

33 g of α-chloro-α-(4-methoxybenzoyl)-2-chloro-5-dodecyloxycarbonylacetanilide and 16 g of 1-(3-carboxyphenyl)-5-mercaptotetrazole were dispersed in 200 ml of chloroform, to which was added dropwise 10 ml of triethylamine at room temperature over a period of about 30 minutes. After stirring for 3 hours the chloroform solution was washed with water and concentrated under reduced pressure. The residue was recrystallized from methanol to obtain 37.4 g of α-(4-methoxybenzoyl)-α-[1-(3-carboxyphenyl)-5-tetrazolylthio]-2-chloro-5-dodecyloxycarbonylacetanilide. Yield: 85%. Melting Point: 155 to 162°C.

Synthesis of 1-acetyl-2-(4-nitrophenyl)hydrazine

200 ml of acetic acid was added to 153 g of p-nitrophenylhydrazine and the mixture was heated on a steam bath for 1 hour with stirring. After cooling to room temperature, the crystals precipitated were collected by filtration, washed with ethyl acetate and dried to obtain 173 g of 1-acetyl-2-(4-nitrophenyl)hydrazine. Yield: 88%. Melting Point: 205.5 to 206°C.

Synthesis of 1-acetyl-2-(4-aminophenyl)hydrazine

To 50 g of 1-acetyl-2-(4-nitrophenyl)hydrazine, were added 1 g of palladium-carbon having about 5% palladium on active carbon and 250 ml of ethanol and the mixture was subjected to catalytic hydrogenation in an autoclave. After removing the catalyst by filtration, the filtrate was cooled and the crystals precipitated were collected by filtration and dried to obtain 36.5 g of 1-acetyl-2-(4-aminophenyl)hydrazine. Yield: 86%. Melting Point: 138 to 143°C.

Synthesis of Compound 1

7.4 g of α-(4-methoxybenzoyl)-α-[1-(3-carboxyphenyl)-5-tetrazolylthio]-2-chloro-5-dodecyloxycarbonylacetanilide and 2 g of 1-acetyl-2-(4-aminophenyl)hydrazine were dissolved in dimethylformamide and to the solution was added dropwise 5 ml of a dimethylamide solution containing 2.1 g of dicyclohexylcarbodiimide under nitrogen atmosphere while cooling at 10°C or below. The mixture was further stirred at room temperature for 2 hours and filtered. To the filtrate was added water

and the filtrate was extracted with ethyl acetate. The ethyl acetate was distilled off under reduced pressure, the residue was crystallized with methanol to obtain 5.6 g of the desired Compound 1. Yield: 63%. Melting Point: 185 to 193°C.

Elementary Analysis for $C_{45}H_{51}N_8O_7ClS$

| | | | |
|---|---|---|---|
| Calcd.: | H: 5.82%; | C: 61.18%; | N: 12.68% |
| Found: | H: 5.76%; | C: 61.11%; | N: 12.77% |

SYNTHESIS EXAMPLE 2

Synthesis of Compound 2

Synthesis of α-pivaloyl-α-[1-(3-carboxyphenyl)-5-tetrazolylthio]-2-chloro-5-dodecyloxycarbonylacetanilide

36.6 g of 1-(3-carboxyphenyl)-5-mercaptotetrazole and 23.1 ml of triethylamine were dispersed in 300 ml of chloroform, to which was added dropwise 200 ml of a chloroform solution containing 54.4 g of α-pivaloyl-α-bromo-2-chloro-5-dodecyloxycarbonylacetanilide at room temperature. After further stirring for 2 hours, the mixture was filtered and washed with water. The chloroform layer was separated, dried with sodium sulfate, concentrated and the residue was crystallized with methanol to obtain 62.6 g of the desired compound. Yield: 91.1%. Melting Point: 145 to 148°C.

Synthesis of Compound 2

Using 20.6 g of α-pivaloyl-α-[1-(3-carboxyphenyl)-5-tetrazolylthio]-2-chloro-5-dodecyloxycarbonyl-

acetanilide, 6.0 g of 1-acetyl-2-(4-aminophenyl)-hydrazine and 6.2 g of dicyclohexylcarbodiimide, the same procedure as described in Synthesis Example 1 was repeated except for crystallizing the residue with 150 ml of ethanol to obtain 15.1 g of the desired Compound 2. Yield: 60.4%. Melting Point: 122 to 124°C.

Elementary Analysis for $C_{42}H_{53}N_8O_6SCl$

| | | | |
|---|---|---|---|
| Calcd.: | H: 6.41%; | C: 60.53%; | N: 13.44% |
| Found: | H: 6.53%; | C: 60.34%; | N: 13.41% |

SYNTHESIS EXAMPLE 3

Synthesis of Compounds 6 and 7

Compound D was synthesized by the following reaction:

Synthesis of 5-benzyloxycarbonylbenzotriazole

81.5 g of 5-carboxybenzotriazole obtained from 3,4-diamino benzoic acid and sodium nitrite (prepared by reacting in acetic acid at about 10°C, yield: about 70 %), 64.8 g of benzyl alcohol and 1 g of 4-dimethylaminopyridine were dissolved in 300 ml of acetonitrile and to the solution was added dropwise 103 g of dicyclohexylcarbodiimide at room temperature. After the completion of the addition, the mixture was stirred for 1 hour and then refluxed by heating for 1 hour. The reaction solution was cooled and filtered to remove dicyclohexylurea. To the filtrate was added 500 ml of water, and it was then extracted with 300 ml of ethyl

acetate. The ethyl acetate layer was washed with water, dried with sodium sulfate and concentrated to obtain 109 g of the desired compound as an oily product. Yield: 86%.

Systhesis of Compound B

63.2 g of Compound A was dissolved in 300 ml of chlorofolm and to the solution was added dropwise 11.6 g of bromine while cooling with ice at 10°C or below. The reaction solution was washed with water and dried with calcium chloride to obtain a chloroform solution of Compound B.

Synthesisof Compound C

30.8 g of 5-benzyloxycarbonylbenzotriazole and 12.3 g of triethylamine were dissolved in 300 ml of chloroform and to the solution was added dropwise the chloroform solution of Compound B (containing 0.061 mol of Compound B) at room temperature. After stirring for 3 hours, the reaction solution was washed with water, dried and concentrated. The residue was separated and purified by silica gel column chromatography to obtain 50.8 g of the desired compound as an oily product. Yield: 73%.

Sythesis of Compound D

To 50.8 g of Compound C and 3.7 g of palladium-carbon was added 300 ml of ethyl acetate and the mixture was subjected to catalytic hydrogenation in an autoclave.

After the completion of the reaction, the catalyst was removed by filtration, and the filtrate was concentrated and crystallized with 200 ml of acetonitrile to obtain 28.1 g of the desired Compound D. Yield: 62%. Melting Point: 116°C.

A

B

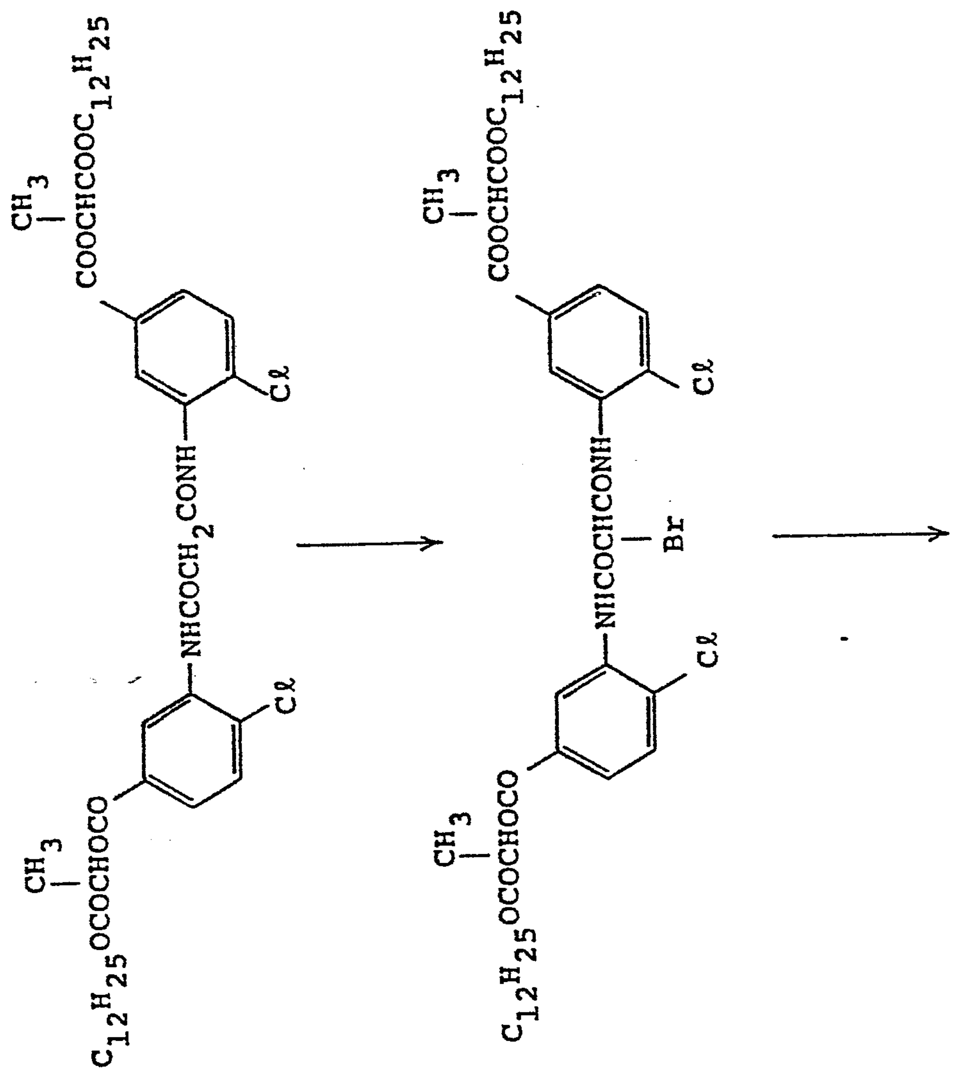

C

D

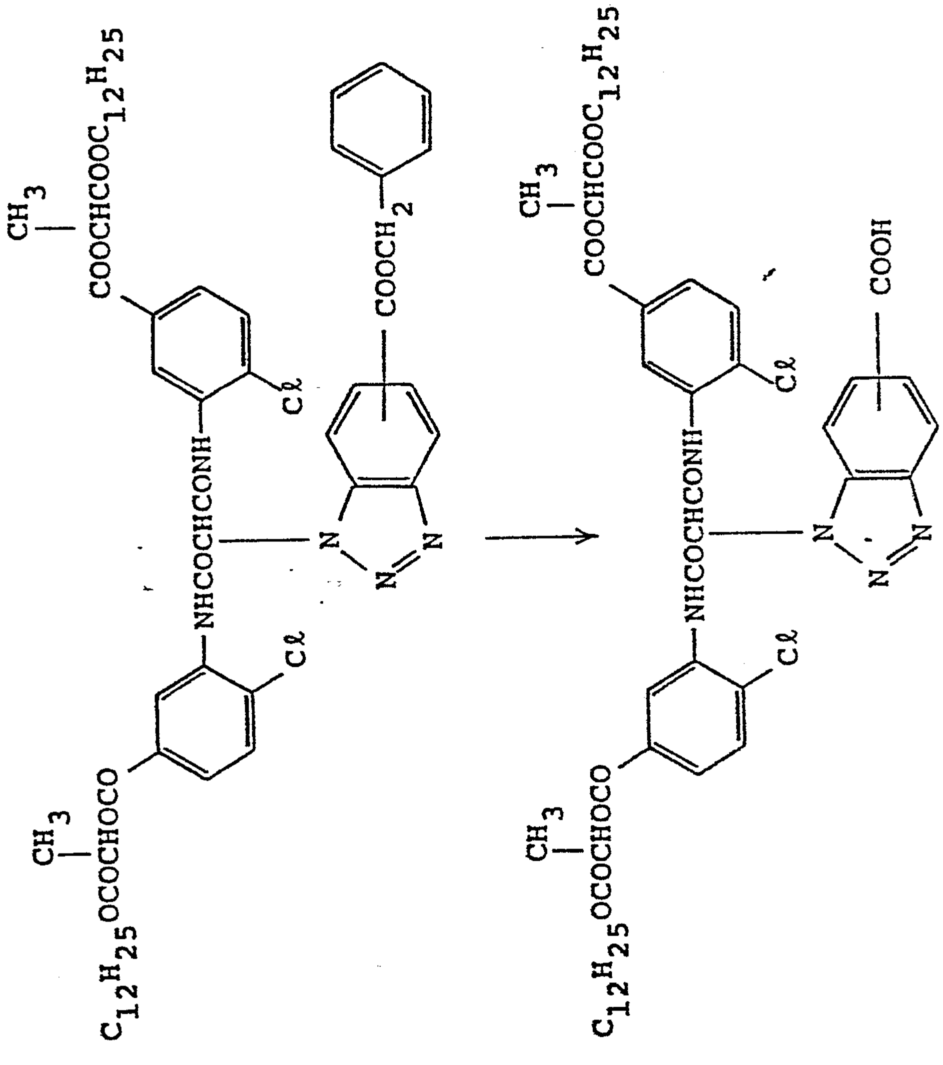

Synthesis of Compound 6

10.5 g of Compound D and 3.3 g of 1-acetyl-2-(4-aminophenyl)hydrazine were dissolved in 150 ml of tetrahydrofuran and to the solution was added dropwise 2.3 g of dicyclohexylcarbodiimide at room temperature. After stirring for 3 hours, the dicyclohexylurea formed was removed by filtration and the filtrate was concentrated. The residue was crystallized with 200 ml of acetonitrile to obtain 5.1 g of the desired Compound 6. Yield: 42.5%. Melting Point: 170 to 178°C.

Elementary Analysis for $C_{62}H_{80}N_8O_{12}Cl_2$

| | | | |
|---|---|---|---|
| Calcd.: | H: 6.72%; | C: 62.05%; | N:9.34% |
| Found: | H: 6.81%; | C: 62.00%; | N: 9.32% |

Synthesis of 1-butanoyl-2-(4-nitrophenyl)hydrazine

36 g of p-nitrophenylhydrazine and 33 ml of triethylamine were dissolved in 150 ml of acetonitrile and to the solution was added dropwise 25 g of butanoyl chloride while cooling with water. After stirring for 1 hour, the reaction mixture was poured into 1 liter of water and the crystals precipitated were collected by filtration, washed with water and dried to obtain 49.1 g of the desired compound. Yield: 94%.

Synthesis of 1-(4-aminophenyl)-2-butanoylhydrazine

To 49.1 g of 1-butanoyl-2-(4-nitrophenyl)-hydrazine and 1 g of palladium-carbon was added 300 ml of tetrahydrofuran and the mixture was subjected to catalytic

hydrogenation in an autoclave. After the completion of the reaction, the catalyst was removed by filtration, and the filtrate was concentrated and crystallized with ethanol to obtain 27.2 g of the desired compound. Yield: 64%.

Synthesis of Compound 7

10.5 g of Compound D and 2.1 g of 1-(4-aminophenyl)-2-butanoylhydrazine were dissolved in 150 ml of tetrahydrofuran and to the solution was added dropwise 2.1 g of dicyclohexylcarbodiimide at room temperature. After stirring for 3 hours, the dicyclohexylurea formed was removed by filtration and the filtrate was concentrated. The residue was crystallized with a mixed solvent of acetonitrile and ethyl acetate to obtain 3.1 g of the desired Compound 7. Yield: 25.2%. Melting Point: 170 to 182°C.

Elementary Analysis for $C_{64}H_{84}N_8O_{12}Cl_2$

| | | | |
|---|---|---|---|
| Calcd.: | H: 6.89%; | C: 62.58%; | N: 9.12% |
| Found: | H: 6.96%; | C: 62.44%; | N: 9.14% |

The couplers according to the present invention can be used in an amount of from 0.001 to 100% by mole, preferably from 0.1 to 10% by mole, based on the total amount of couplers used in an emulsion layer. The total amount of couplers used can be in the range of from $2 \times 10^{-3}$ to $5 \times 10^{-1}$, preferably from $1 \times 10^{-2}$ to $5 \times 10^{-1}$, per mole of silver.

The couplers according to the present invention can also be incorporated into light-insensitive layers of the light-sensitive material and can be combined with other couplers, in an amount of from $10^{-8}$ to $10^{-2}$ mole/$m^2$, preferably from $10^{-6}$ to $10^{-3}$ mole/$m^2$.

The couplers according to the present invention can be used in an amount as small as 1 mol% or less of the total coupler content of an emulsion layer and are advantageous with respect to low fog formation because they have the adsorbing group represented by AD.

The couplers according to the present invention and other couplers as described hereinafter can be incorporated into light-sensitive or light-insensitive layers by known methods, including those described, e.g., in U.S. Patent 2,322,027. For example, the couplers can be dissolved in a solvent and then dispersed in a hydrophilic colloid. Examples of solvents usable for this process include organic solvents having a high boiling point, such as alkyl esters of phthalic acid (e.g., dibutyl phthalate, dioctyl phthalate, etc.), phosphates (e.g., diphenyl phosphate, triphenyl phosphate, tricresyl phosphate, dioctyl butyl phosphate, etc.), citrates (e.g., tributyl acetyl citrate, etc.), benzoates (e.g., octyl benzoate, etc.), alkylamides (e.g., diethyl laurylamides, etc.), esters of fatty acids (e.g., dibutoxyethyl succinate, dioctyl azelate, etc.)

and trimesates (e.g., tributyl trimesate, etc.); and organic solvents having a boiling point of from about 30 to about 150°C, such as lower alkyl acetates (e.g., ethyl acetate, butyl acetate, etc.), ethyl propionate, secondary butyl alcohol, methyl isobutyl ketone, β-ethoxyethyl acetate, methyl cellosolve acetate, or the like. Mixtures of organic solvents having a high boiling point and organic solvents having a low boiling point can also be used.

It is also possible to utilize the dispersing method using polymers described in Japanese Patent Publication No. 39853/76 and Japanese Patent Application (OPI) No. 59943/76.

Couplers having an acid group, such as a carboxylic acid group or a sulfonic acid group, can be introduced into hydrophilic colloids in aqueous alkaline solution.

The photographic emulsion layers of the photographic lghht-sensitive materials of the present invention in addition to the compounds according to the present invention can contain conventional dye forming couplers, i.e., compounds capable of forming color upon oxidative coupling with aromatic primary amine developing agents (e.g., phenylenediamine derivatives, aminophenol derivatives, etc.) during the course of color development processing. Examples of such couplers include magenta

couplers, such as 5-pyrazolone couplers, pyrazolobenzimidazole couplers, cyanoacetylcoumarone couplers and open chain acylacetonitrile couplers, etc.; yellow couplers, such as acylacetamide couplers (e.g., benzoylacetanilides, pivaloylacetanilides, etc.), etc.; and cyan couplers, such as naphthol couplers and phenol couplers, etc. It is preferable to use non-diffusible couplers containing a hydrophobic group ("ballast group") within the molecule or polymeric couplers. They may be either 4-equivalent or 2-equivalent with respect to silver ions. It is also possible to use colored couplers capable of providing color correction, or couplers capable of releasing development inhibitors during the course of development ("DIR couplers").

Further, the emulsion layers may contain non-color-forming DIR coupling compounds which release a development inhibitor, forming a product by a coupling reaction, other than DIR couplers.

Moreover, the photographic light-sensitive material may contain compounds other than DIR couplers which release a development inhibitor during the course of development.

Two or more of the above-described couplers can be incorporated in the same layer to provide the properties required in the photographic light-sensitive material, or the same compound can be added to two or

more layers.

It is advantageous to select photographic color couplers to be used so as to provide images of medium scale. It is preferred that cyan dyes formed from cyan color forming couplers have maximum absorption bands in the wavelength range from about 600 nm to 720 nm; that magenta dyes formed from magenta color forming couplers have maximum absorption bands in the wavelength range from about 500 nm to 580 nm; and the yellow dyes formed from yellow color forming couplers have maximum absorption bands in the wavelength range from about 400 nm to 480 nm.

As the binder or the protective colloid for the photographic emulsion layers or intermediate layers of the photographic light-sensitive material of the present invention, gelatin is advantageously used, but other hydrophilic colloids can be used.

For example, it is possible to use proteins such as gelatin derivatives, graft polymers of gelatin and other high molecules, albumin, casein, etc.; saccharides including cellulose derivatives such as hydroxyethyl cellulose, carboxymethyl cellulose, cellulose sulfate, etc.; sodium alginate, starch derivatives, etc.; and various synthetic hydrophilic high molecular substances including homopolymers or copolymers, for example, polyvinyl alcohol, polyvinyl alcohol semiacetal, poly-N-vinylpyrrolidone, polyacrylic acid, polymethacrylic acid, polyacrylamide, polyvinylimidazole, polyvinylpyrazole, etc.

As gelatin, not only lime-processed gelatin, but also acid-processed gelatin and enzyme-processed gelatin as described in Bull. Soc. Sci. phot. Japan, No. 16, page 30 (1966) may be used. Further, hydrolyzed products or enzymatic products of gelatin can also be used.

In the photographic emulsion layers of the photographic light-sensitive material used in the present invention, any of silver bromide, silver iodobromide, silver iodochlorobromide, silver chlorobromide and silver chloride may be used as the silver halide. Useful silver halide emulsions are those containing 60 mol% or more of silver bromide, 30 mol% or less of silver chloride and 40 mol% or less of silver iodide. A silver iodobromide emulsion containing from 2 to 25 mol% of silver iodide is preferred and a silver iodobromide emulsion containlng from, 8 to 25 mol% of silver iodide is particularly preferred.

Although the mean grain size of silver halide particles in the photographic emulsion (the mean grain size being defined by grain diameter in those particles which are spherical or nearly spherical, and by edge length in those particles which are cubic and

expressed as a mean value calculated from projected areas) is not particularly limited, it is preferably 0.6 μm or more, more preferably 1.0 μm or more and particularly preferably 1.5 μm or more in the silver halide emulsion into which the compound according to the present invention is incorporated.

The distribution of grain size may be broad or narrow.

Silver halide particles in the photographic emulsion may have a regular crystal structure, e.g., a cubic or octahedral structure, an irregular crystal structure, e.g., a spherical or tabular structure, or a composite structure thereof. In addition, silver halide particles composed of grains having different crystal structures may be used.

Further, photographic emulsions wherein at least 50 percent of the total projected area of silver halide particles comprises tabular silver halide grains having a diameter at least five times their thickness may be employed.

The inner portion and the surface layer of the silver halide grains may be different in phase or may be of the same phase. The silver halide particles may form a latent image mainly on the surface thereof, or form latent image mainly in the interior thereof.

Photographic emulsions used in the present

invention can be prepared in any suitable manner, e.g., by the methods as described in P. Glafkides, Chimie et Physique Photographique, Paul Montel (1967), G.F. Duffin, Photographic Emulsion Chemistry, The Focal Press (1966), and V.L. Zelikman et al., Making and Coating Photographic Emulsion, The Focal Press (1964), including an acid process, a neutral process and an ammonia process.

Soluble silver salts and soluble halogen salts can be reacted by techniques such as a single jet process, a double jet process, and a combination thereof. In addition, a "reversal mixing process" can be used in which silver halide particles are formed in the presence of an excess of silver ions.

As one double jet process, the "controlled double jet process", in which the pAg in a liquid phase where silver halide is formed is maintained at a predetermined level, can be employed to produce a silver halide emulsion in which the crystal form is regular and the grain size is nearly uniform.

Two or more kinds of silver halide emulsions which are prepared separately may be used as a mixture.

The formation or physical ripening of silver halide particles may be carrled out in tne presence cadmium salts, zinc salts, lead salts, thallium salts, iridium salts or iridium complex salts, rhodium salts or rhodium complex salts or ion salts or ion complex salts.

For removal of soluble salts from the emulsion after precipitate formation or physical ripening, a noodle washing process in which gelatin is gelated may be used as well as a flocculation process employing inorganic salts having a polyvalent anion (e.g., sodium sulfate), anionic surface active agents, anionic polymers (e.g., polystyrenesulfonic acid), or gelatin derivatives (e.g., aliphatic acylated gelatin, aromatic acylated gelatin and aromatic carbamoylated gelatin).

Silver halide emulsions are usually chemically Sensitized. For this chemical sensitization, for example, the methods described in H. Frieser ed., Die Grundlagen der Photographischen Prozesse mit Silberhalogeniden, Akademische Verlagsgesselschaft, pages 675 to 734 (1968) can be used. Namely, a sulfur sensitization process using active gelatin or compounds (e.g., thiosulfates, thioureas, mercapto compounds and rhodanines) containing sulfur capable of reacting with silver; a reduction sensitization process using reducing substances (e.g., stannous salts, amines, hydrazine derivatives, formamidinesulfinic acid, and silane compounds; a noble metal sensitization process using noble metal compounds (e.g., complex salts of Group VIII metals in the Periodic Table, such as Pt, Ir and Pd, etc., as well as gold complex salts); and so forth can be applied alone or in combination with each other. Sensitization process, for

example, in U.S. Patents 2,399,083, 2,448,060, and British Patent 618,061.

Photographic emulsions as used in the present invention may include various compounds for the purpose of preventing fog formation or of stabilizing photographic performance in light-sensitive material during the production, storage or photographic processing thereof. For example, antifoggants or stabilizers can be incorporated, including azoles, such as benzothiazolium salts, nitroimidazoles, nitrobenzimidazoles, chlorobenzimidazoles, bromobenzimidazoles, mercaptothiazoles, mercaptobenzothiazoles, mercaptobenzimidazoles, mercaptothiadiazoles, aminotriazoles, benzotriazoles, nitrobenzotriazoles, mercaptotetrazoles (particularly 1-phenyl-5-mercaptotetrazole), etc.; mercaptopyrimidines; mercaptotriazines; thioketo compounds, such as oxazolinethione, etc.; azaindenes, such as triazaindenes, tetraazaindenes (particularly 4-hydroxy-substituted (1,3,3a,7)tetraazaindenes), pentaazaindenes, etc.; benzenethiosulfonic acids; benzenethiosulfinic acids; benzenesulfonic amides; etc.

Specific examples and methods of using antifoggants and stabilizers are described in U.S. Patents 3,954,474 and 3,982,947 and Japanese Patent Publication No. 28660/77.

Photographic emulsion layers or other

hydrophilic colloid layers of the light-sensitive material of the present invention can contain various surface active agents as coating aids or for other various purposes, e.g., prevention of charging, improvement of slipping properties, acceleration of emulsification and dispersion, prevention of adhesion, and improvement of photographic characteristics (particularly development acceleration, high contrast, and sensitization).

Surface active agents which can be used include nonionic surface active agents, e.g., saponin (steroid-based), alkylene oxide derivatives (e.g., polyethylene glycol, a polyethylene glycol/polypropylene glycol condensate, polyethylene glycol alkyl ethers or polyethylene glycol alkylaryl ethers, polyethylene glycol esters, polyethylene glycol sorbitan esters, polyalkylene glycol alkylamines or polyalkylene glycol alkylamides, and silicone/polyethylene oxide adducts, etc.), glycidol derivatives (e.g., alkenylsuccinic acid polyglyceride and alkylphenol polyglyceride, etc.), fatty acid esters of polyhydric alcohols, and alkyl esters of sugar, etc.; anionic surface active agents containing acidic groups, such as a carboxy group, a sulfo group, a phospho group, a sulfuric acid ester group, and a phosphoric acid ester group, for example, alkylcarboxylic acid salts, alkylsulfonic acid salts, alkylbenzenesulfonic acid salts, alkylnaphthalenesulfonic acid salts, alkylsulfuric acid

esters, alkylphosphoric acid esters, N-acyl-N-alkyltaurines, sulfosuccinic acid esters, sulfoalkylpolyoxyethylene alkylphenyl ethers, and polyoxyethylene alkylphosphoric acid esters; amphoteric surface active agents, such as amino acids, aminoalkylsulfonic acids, aminoalkylsulfuric acid or aminoalkylphosphoric acid esters, alkylbetaines, and amine oxides; and cationic surface active agents, e.g., alkylamine salts, aliphatic or aromatic quaternary ammonium salts, heterocyclic quaternary ammonium salts (e.g., pyridinium and imidazolium), and aliphatic or heterocyclic phosphonium or sulfonium salts.

The photographic emulsion layer of the photographic light-sensitive material of the present invention may contain compounds such as polyalkylene oxide or its ether, ester, amines,thioether compounds, thiomorpholines, quaternary ammonium salt compounds, urethane derivatives, urea derivatives, imidazole derivatives, and 3-pyrazolidones for the purpose of increasing sensitivity or contrast, or of accelerating development. For example, the compounds described in U.S. Patents 2,400,532, 2,423,549, 2,716,062, 3,617,280, 3,772,021 and 3,808,003, British Patent 1,488,991, etc. can be used.

Photographic emulsion layers or other hydrophilic colloid layers of the photographic light-sensitive material of the present invention can include water-insoluble or sparingly soluble synthetic polymer dispersions for the purpose of improving dimensional stability, etc. Synthetic polymers which can be used include homo- or copolymers of alkyl acrylate or methacrylate, alkoxyalkyl acrylate or methacrylate, glycidyl acrylate or methacrylate, acrylamide or methacrylamide, vinyl esters (e.g., vinyl acetate), acrylonitrile, olefins, styrene, etc. and copolymers of these monomers and acrylic acid, methacrylic acid, α,β-unsaturated dicarboxylic acid, hydroxyalkyl acrylate or methacrylate, sulfoalkyl acrylate or methacrylate, and styrenesulfonic acid.

In photographic processing of layers composed of photographic emulsions in the photographic light-sensitive material of the present invention, any known procedure and known processing solutions, e.g., those described in Research Disclosure, No. 176, pages 28 to 30 can be used. This photographic processing may be a color photographic process to form dye images, if desired. The processing temperature is usually between 18°C and 50°C, although it may be lower than 18°C or higher than 50°C, if desired.

In a typical developing technique, a developing agent is incorporated in a light-sensitive material, for example, in an emulsion layer, and the light-sensitive material is developed in an alkaline aqueous solution.

Hydrophobic developing agents can be incorporated by various methods such as those described in Research Disclosure, No. 169 (RD-16928), U.S. Patent 2,739,890, British Patent 813,253, West German Patent 1,547,763, etc. This photographic processing may be performed in combination with a treatment for stabilizing silver salts using thiocyanic acid salts.

Any conventional fixing solutions can be used in the present invention, containing such fixing agents as thiosulfuric acid salts and thiocyanic acid salts, as well as organic sulfur compounds which are effective as fixing agents can be used. These fixing solutions may contain water-soluble aluminum salts as hardeners.

Formation of dye images can be achieved by any conventional method, including, for example, the negative-poositive method described in Journal of the Society of Motion Picture and Television Engineers, Vol. 61, 667 - 701 (1953).

Color developing solutions are usually alkaline aqueous solutions containing color developing agents such as known primary aromatic amine developing agents, e.g., phenylenediamines such as 4-amino-N,N-diethylaniline, 3-methyl-4-amino-N,N-diethylaniline, 4-amino-N-ethyl-N-β-hydroxyethylaniline, 3-methyl-4-amino-N-ethyl-N-β-hydroxyethylaniline, 3-methyl-4-amino-N-ethyl-N- -methanesulfonamidoethylaniline, and 4-amino-3-methyl-

N-ethyl-N-β-methoxyethylaniline.

In addition, the compounds as described in L.F.A. Mason, Photographic Processing Chemistry, Focal Press, pages 226 to 229 (1966), U.S. Patents 2,193,015 and 2,592,364, Japanese Patent Application (OPI) No. 64933/73, etc., may beused.

The color developing solutions can further contain pH buffering agents, such as sulfites, carbonates, borates and phosphates of alkali metals, developing inhibitors or anti-fogging agents such as bromides, iodides or organic anti-fogging agents, etc. If desired, the color developing solution can also contain water softeners, preservatives such as hydroxylamine; organic solvents such benzyl alcohol, diethylene glycol, etc.; developing accelerators such as polyethylene glycol, quaternary ammonium salts, amines; dye forming couplers; competing couplers; fogging agents such as sodium borohydride; auxiliary developing agents such as 1-phenyl-3-pyrazolidone; viscosity-imparting agents; polycarboxylic acid type chelating agents; anti-oxidizing agents; and the like.

Specific examples of such additives are described in Research Disclosure, Vol. 176, No. 17643, U.S. Patent 4,083,723, and West German Patent (OLS) No. 2,622,950, etc.

After color development, the photographic

emulsion layer is usually bleached. This bleach processing may be performed simultaneously with a fix processing, or it may be performed independently.

Bleaching agents which can be used include compounds of polyvalent metals, e.g., iron (III), cobalt (III), chromium (VI), and copper (II), peracids, quinones and nitroso compounds. For example, ferricyanides; dichromates; organic complex salts of iron (III) or cobalt (III), e.g., complex salts of organic acids, such as aminopolycarboxylic acids (e.g., ethylenediaminetetraacetic acid, nitrilotriacetic acid, 1,3-diamino-2-propanoltetraacetic acid, etc.) or organic acids (e.g., citric acid, tartaric acid, malic acid, etc.); persulfates; permanganates; nitrosophenol, etc. can be used. Of these compounds, potassium ferricyanide, iron (III) sodium ethylenediaminetetraacetate, and iron (III) ammonium ethylenediaminetetraacetate are particularly useful. Ethylenediaminetetraacetic acid iron (III) complex salts are useful in both a separate bleaching solution and a mono-bath bleach-fixing solution.

Bleaching or bleach-fixing solutions can contain various conventional additives, such as bleach accelerating agents described in U.S. Patents 3,042,520 and 3,241,966, Japanese Patent Publication Nos. 8506/70 and 8836/70, etc., and thiol compounds described in Japanese Patent Application (OPI) No. 65732/78.

The photographic emulsion used in the present invention can also be spectrally sensitized with methine dyes or other dyes, such as cyanine dyes, merocyanine dyes, complex cyanine dyes, complex merocyanine dyes, holopolar cyanine dyes, hemicyanine dyes, styryl dyes, and hemioxonol dyes. Of these dyes, cyanine dyes, merocyanine dyes and complex merocyanine dyes are particularly useful. Any conventional nuclei for cyanine dyes may be used in these dyes as basic heterocyclic nuclei, including, e.g., a pyrroline nucleus, an oxazoline nucleus, a thiazoline nucleus, a pyrrole nucleus, an oxazole nucleus, a thiazole nucleus, a selenazole nucleus, an imidazole nucleus, a tetrazole nucleus, a pyridine nucleus, etc., in addition to nuclei formed by condensing alicyclic hydrocarbon rings with these nuclei and nuclei formed by condensing aromatic hydrocarbon rings with these nuclei, e.g., an indolenine nucleus, a benzindolenine nucleus, an indole nucleus, a benzoxazole nucleus, a naphthoxazole nucleus, a benzothiazole nucleus, a naphthothiazole nucleus, a benzoselenazole nucleus, a benzimidazole nucleus and a quinoline nucleus. The carbon atoms of these nuclei can also be substituted.

The merocyanine dyes and the complex merocyanine dyes that can be employed contain 5- or 6-membered heterocyclic nuclei such as a pyrazolin-5-one

nucleus, a thiohydantoin nucleus, a 2-thioxazolidin-2,4-dione nucleus, a thiazolidine-2,4-dione nucleus, a rhodanine nucleus, a thiobarbituric acid nucleus, and the like.

These sensitizing dyes can be employed individually, and can also be employed in combination. A combination of sensitizlng dyes is often used particularly for the purpose of supersensitization, and representative examples are described in U.S. Patents 2,688,545, 2,977,229, 3,397,060, 3,522,052, 3,527,641, 3,617,293, 3,628,964, 3,666,480, 3,672,898, 3,679,428, 3,703,377, 3,769,301, 3,814,609, 3,837,862 and 4,026,707, British Patents 1,344,281 and 1,507,803, Japanese Patent Publication Nos. 4936/68 and 12375/78, and Japanese Patent Application (OPI) Nos. 110618/77 and 109925/77.

The sensitizing dyes may be present in the emulsion together with dyes which themselves do not have spectrally sensitizing effects but which exhibit a supersensitizing effect, or materials which do not substantially absorb visible light but which exhibit a supersensitizing effect. For example, aminostilbene compounds substituted with a nitrogen-containinq heterocvclic ring qroup (e.g., those described in U.S. Patents 2,933,390 and 3,635,721), aromatic organic acid-formaldehyde condensates (e.g., those described in U.S. Patent 3,743,510), cadmium salts, azaindene compounds,

and the like, can be used. The combinations described in U.S. Patents 3,615,613, 3,615,641, 3,617,295 and 3,635,721 are particularly useful.

The present invention is also applicable to a multilayer multicolor photographic material containing layers sensitive to at least two different spectral wavelength ranges on a support. A multi layer natural color photographic material generally possesses at least one red-sensitive silver halide emulsion layer, at least one green-sensitive silver halide emulsion layer and at least one blue-sensitive silver halide emulsion layer on a support. The order of these layers can be varied, if desired. Ordinarily, a cyan forming coupler is present in a red-sensitive emulsion layer, a magenta forming coupler is present in a green-sensitive emulsion layer and a yellow forming coupler is present in a blue-sensitive emulsion layer, although, if desired, a different combination can be employed.

The photographic light-sensitive material of the present invention may contain inorganic or organic hardeners in the photographic emulsion layers and other hydrophilic colloid layers thereof. For example, chromium salts (e.g., chromium alum, chromium acetate, etc.), aldehydes (e.g., formaldehyde, glyoxal, glutaraldehyde, etc.), N-methylol compounds (e.g., dimethylolurea, methyloldimethylhydantoin, etc.), dioxane derivatives

(e.g., 2,3-dihydroxydioxane, etc.), active vinyl compounds (e.g., 1,3,5-triacryloylhexahydro-s-triazine, 1,3-vinylsulfonyl-2-propanol, etc.), active halogen compounds (e.g., 2,4-dichloro-6-hydroxy-s-triazine, etc.), and mucohalogenic acids (e.g., mucochloric acid, mucophenoxychloric acid, etc.) can be used alone or in combination with each other.

In the photographic light-sensitive material of the invention, when dyes, ultraviolet light absorbing agents, and the like are incorporated in the hydrophilic colloid layers, they may be mordanted, for example, with cationic polymers.

The photographic light-sensitive material of the present invention may contain color fog preventing agents including hydroquinone derivatives, aminophenol derivatives, gallic acid derivatives and ascorbic acid derivatives.

The hydrophilic colloid layers of the light-sensitive material of the present invention can contain ultraviolet light absorbing agents. For example, benzotriazole compounds substituted with aryl groups (e.g., those described in U.S. Patent 3,533,794), 4-thiazolidone compounds (e.g., those described in U.S. Patents 3,314,794 and 3,352,681), benzophenone compounds (e.g., those described in Japanese Patent Application (OPI) No. 2784/71), cinnamic acid ester compounds (e.g.,

those described in U.S. Patents 3,707,375 and 3,705,805), butadiene compounds (e.g., those described in U.S. Patent 4,045,229) or benzoxazole compounds (e.g., those described in U.S. Patent 3,700,455) can be employed, as well as the compounds described in U.S. Patent 3,499,762, Japanese Patent Application (OPI) No. 48535/79. Ultraviolet light absorbing couplers (e.g., α-naphthol type cyan color forming couplers) and ultraviolet light absorbing polymers can also be employed. These ultraviolet light absorbing agents can also be mordanted in a specific layer(s), if desired.

The photographic light-sensitive material of the present invention may contain water-soluble dyes in its hydrophilic colloid layers as filter dyes or for various purposes, e.g., irradiation prevention. Examples of such dyes include oxonol dyes, hemioxonol dyes, styryl dyes, merocyanine dyes, cyanine dyes, and azo dyes. In particular, oxonol dyes, hemioxonol dyes, and merocyanine dyes are useful.

In the present invention, known color fading preventing agents can be used together and color image stabilizers can be used alone or in combination with each other. Typical known color fading preventing agents include hydroquinone derivatives, gallic acid derivatives, p-alkoxyphenols, p-oxyphenol derivatives and bisphenols.

The present invention will be explained in

greater detail with reference to the following examples, but the present invention should not be construed as being limited thereto. Unless otherwise indicated, all parts, percents and ratios are by weight.

EXAMPLE 1

In order to evaluate the effectiveness of the compounds according to the present invention, a multilayer color photographic light-sensitive material with layers having the compositions described below, which are designated Sample 101, was prepared. The coating amounts of silver halide emulsions are indicated with reference to the amounts of silver coated.

Sample 101

First Layer: Antihalation Layer

A gelatin layer containing

| | |
|---|---|
| Black colloidal silver | 0.18 $g/m^2$ |
| Ultraviolet Light Absorbing Agent C-1 | 0.12 $g/m^2$ |
| Ultraviolet Light Absorbing Agent C-2 | 0.17 $g/m^2$ |

Second Layer: Intermediate Layer

A gelatin layer containing

| | |
|---|---|
| 2,5-Di-tert-pentadecylhydroquinone | 0.18 $g/m^2$ |
| Coupler C-3 | 0.11 $g/m^2$ |
| Silver iodobromide emulsion (iodide content: 1 mol%, mean grain size: 0.07 μ) | 0.15 $g/m^2$ |

Third Layer: First Red-Sensitive Emulsion Layer

A gelatin layer containing

| | | |
|---|---|---|
| Silver iodobromide emulsion (iodide content: 6 mol%, mean grain size: 0.6 μ) | | 0.72 $g/m^2$ |
| Sensitizing Dye I | $7.0 \times 10^{-5}$ | mol per mol of silver |
| Sensitizing Dye II | $2.0 \times 10^{-5}$ | mol per mol of silver |
| Sensitizing Dye III | $2.8 \times 10^{-4}$ | mol per mol of silver |
| Sensitizing Dye IV | $2.0 \times 10^{-5}$ | mol per mol of silver |
| Coupler C-4 | | 0.093 $g/m^2$ |
| Coupler C-5 | | 0.31 $g/m^2$ |
| Coupler C-6 | | 0.010 $g/m^2$ |

Fourth Layer: Second Red-Sensitive Emulsion Layer

A gelatin layer containing

Silver iodobromide emulsion (iodide content: 10 mol%, mean grain size: 1.5 μ) 1.6 $g/m^2$

Sensitizing Dye I $5.2x10^{-5}$ mol per mol of silver

Sensitizing Dye II $1.5x10^{-5}$ mol per mol of silver

Sensitizing Dye III $2.1x10^{-4}$ mol per mol of silver

Sensitizing Dye IV $1.5x10^{-5}$ mol per mol of silver

Coupler C-4 0.10 $g/m^2$

Coupler C-5 0.061 $g/m^2$

Coupler C-6 0.005 $g/m^2$

Coupler C-7 0.046 $g/m^2$

Fifth Layer: Third Red-Sensitive Emulsion Layer

A gelatin layer containing

Silver iodobromide emulsion (iodide content: 12 mol%, mean grain size: 2.2 μ) 1.6 $g/m^2$

Sensitizing Dye I $5.5x10^{-5}$ mol per mol of silver

Sensitizing Dye II $1.6x10^{-5}$ mol per mol of silver

Sensitizing Dye III $2.2x10^{-5}$ mol per mol of silver

Sensitizing Dye IV $1.6x10^{-5}$ mol per mol of silver

Coupler C-5 0.050 $g/m^2$

Sixth Layer: Intermediate Layer

A gelatin layer

Seventh Layer: First Green-Sensitive Emulsion Layer

A gelatin layer containing

Silver iodobromide emulsion (iodide content: 5 mol%, mean grain size: 0.5 μ) 0.55 $g/m^2$

| | | |
|---|---|---|
| Sensitizing Dye V | $3.8x10^{-4}$ mol per mol of silver | |
| Sensitizing Dye VI | $3.0x10^{-5}$ mol per mol of silver | |
| Sensitizing Dye VII | $1.2x10^{-4}$ mol per mol of silver | |
| Coupler C-8 | | 0.29 $g/m^2$ |
| Coupler C-9 | | 0.040 $g/m^2$ |
| Coupler C-10 | | 0.055 $g/m^2$ |
| Coupler C-11 | | 0.058 $g/m^2$ |

Eighth Layer: Second Green-Sensitive Emulsion Layer

A gelatin layer containing

| | | |
|---|---|---|
| Silver iodobromide emulsion (iodide content: 6 mol%, mean grain size: 1.5 μ) | | 1.5 $g/m^2$ |
| Sensitizing Dye V | $2.7x10^{-4}$ mol per mol of silver | |
| Sensitizing Dye VI | $2.1x10^{-5}$ mol per mol of silver | |
| Sensitizing Dye VII | $8.5x10^{-5}$ mol per mol of silver | |
| Coupler C-8 | | 0.25 $g/m^2$ |
| Coupler C-9 | | 0.013 $g/m^2$ |
| Coupler C-10 | | 0.009 $g/m^2$ |
| Coupler C-11 | | 0.011 $g/m^2$ |

Ninth Layer: Third Green-Sensitive Emulsion Layer

A gelatin layer containing

| | | |
|---|---|---|
| Silver iodobromide emulsion (iodide content: 12 mol%, mean grain size: 2.2 μ) | | 1.5 $g/m^2$ |
| Sensitizing Dye V | $3.0x10^{-4}$ mol per mol of silver | |
| Sensitizing Dye VI | $2.4x10^{-5}$ mol per mol of silver | |
| Sensitizing Dye VII | $9.5x10^{-5}$ mol per mol of silver | |

| | |
|---|---|
| Coupler C-12 | 0.070 $g/m^2$ |
| Coupler C-11 | 0.002 $g/m^2$ |
| Coupler C-9 | 0.013 $g/m^2$ |

Tenth Layer: Yellow Filter Layer

A gelatin layer containing

| | |
|---|---|
| Yellow colloidal silver | 0.04 $g/m^2$ |
| 2,5-Di-tert-pentadecylhydroquinone | 0.31 $g/m^2$ |

Eleventh Layer: First Blue-Sensitive Emulsion Layer

A gelatin layer containing

| | |
|---|---|
| Silver iodobromide emulsion (iodide content: 6 mol%, mean grain size: 0.4 μ) | 0.32 $g/m^2$ |
| Coupler C-13 | 0.68 $g/m^2$ |
| Coupler C-14 | 0.030 $g/m^2$ |

Twelfth Layer: Second Blue-Sensitive Emulsion Layer

A gelatin layer containing

| | |
|---|---|
| Silver iodobromide emulsion (iodide content: 10 mol%, mean grain size: 1.0 μ) | 0.40 $g/m^2$ |
| Coupler C-13 | 0.22 $g/m^2$ |

Sensitizing Dye VIII $2.2 \times 10^{-4}$ mol per mol of silver

Thirteenth Layer: Fine Grain Emulsion Layer

A gelatin layer containing

| | |
|---|---|
| Silver iodobromide emulsion (iodide content: 2 mol%, mean grain size: 0.15 μ) | 0.25 $g/m^2$ |

Fourteenth Layer: Third Blue-Sensitive Emulsion Layer

A gelatin layer containing

| | |
|---|---|
| Silver iodobromide emulsion (iodide content: 10 mol%, mean grain size: 2.3 μ) | 1.00 $g/m^2$ |
| Coupler C-13 | 0.19 $g/m^2$ |
| Coupler C-15 | 0.5 $mg/m^2$ |
| Sensitizing Dye VIII | $2.3x10^{-4}$ mol per mol of silver |

Fifteenth Layer: First Protective Layer

A gelatin layer containing

| | |
|---|---|
| Ultraviolet Light Absorbing Agent C-1 | 0.14 $g/m^2$ |
| Ultraviolet Light Absorbing Agent C-2 | 0.22 $g/m^2$ |

Sixteenth Layer: Second Protective Layer

A gelatin layer containing

| | |
|---|---|
| Polymethyl methacrylate particles (diameter: 1.5 μ) | 0.05 $g/m^2$ |
| Silver iodobromide emulsion (iodide content: 2 mol%, mean grain size: 0.07 μ) | 0.30 $g/m^2$ |

A gelatin hardener C-16 and a surface active agent were incorporated into each of the layers in addition to the above-described components.

The compounds used for preparing the sample are as follows:

Compunds

C – 1

Polymer of

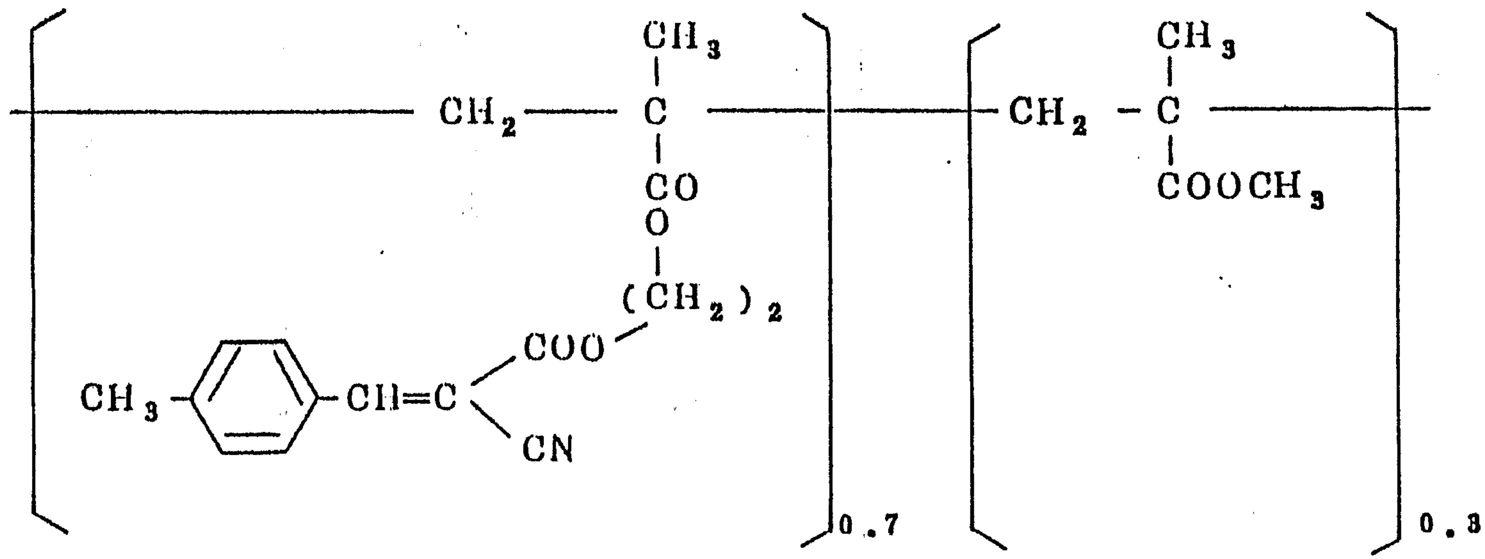

(molecular weight: about 20,000)

C - 2

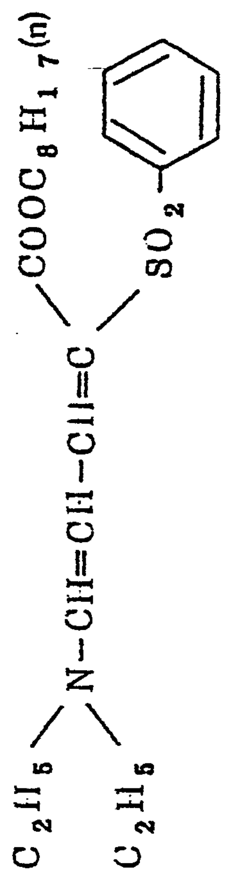

C - 3

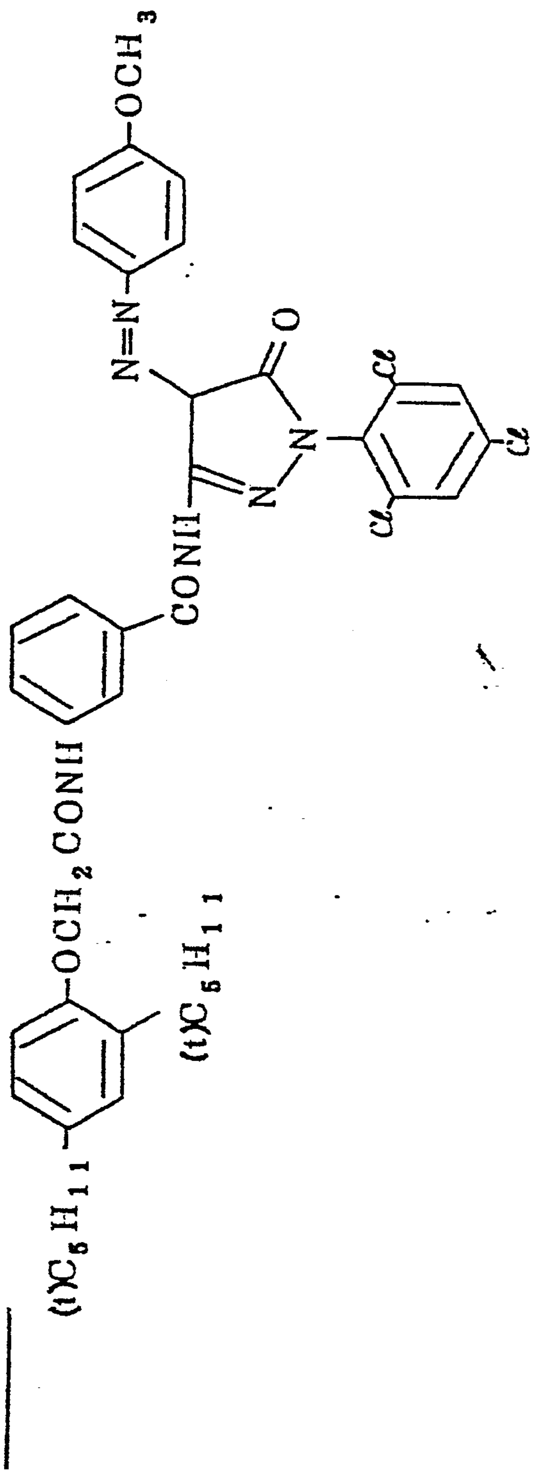

C – 4

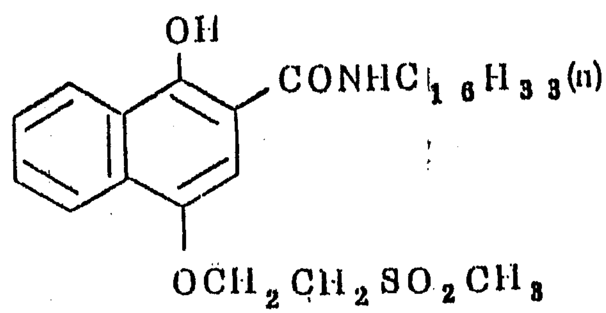

C – 5

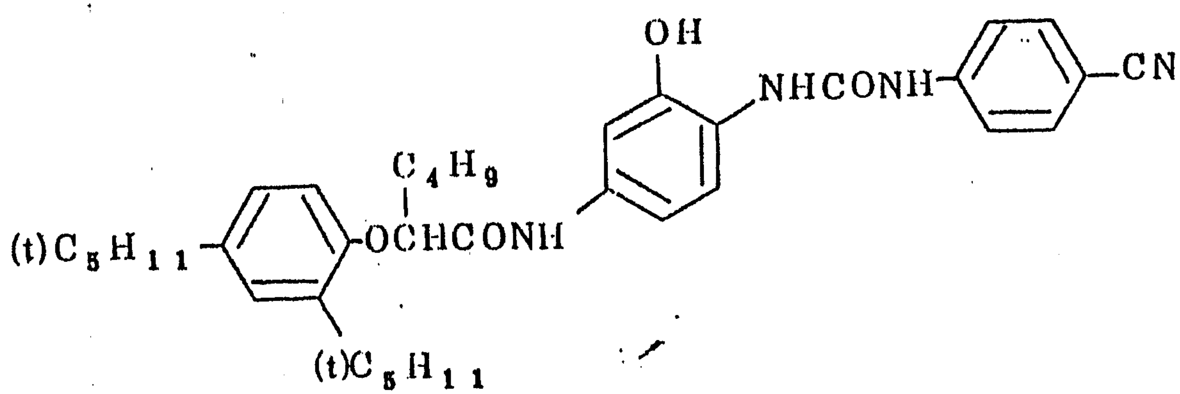

C - 6

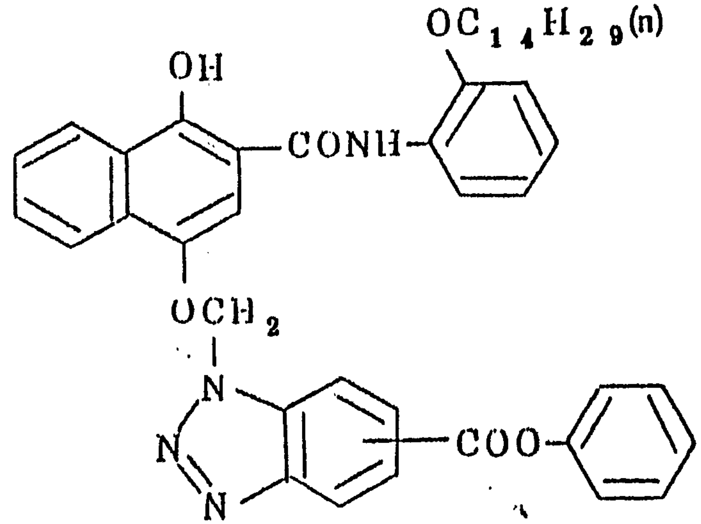

C - 7

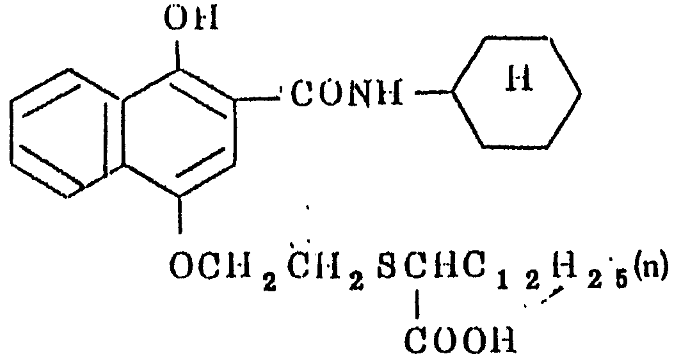

C – 8

Polymer of

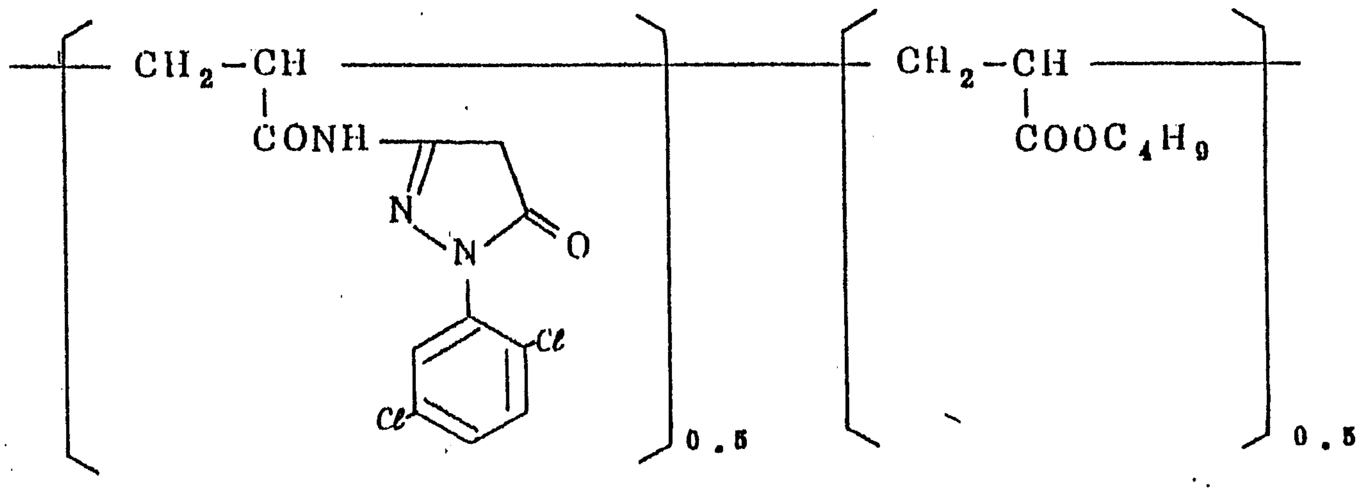

(moleuclar weight: about 25,000)

C - 9

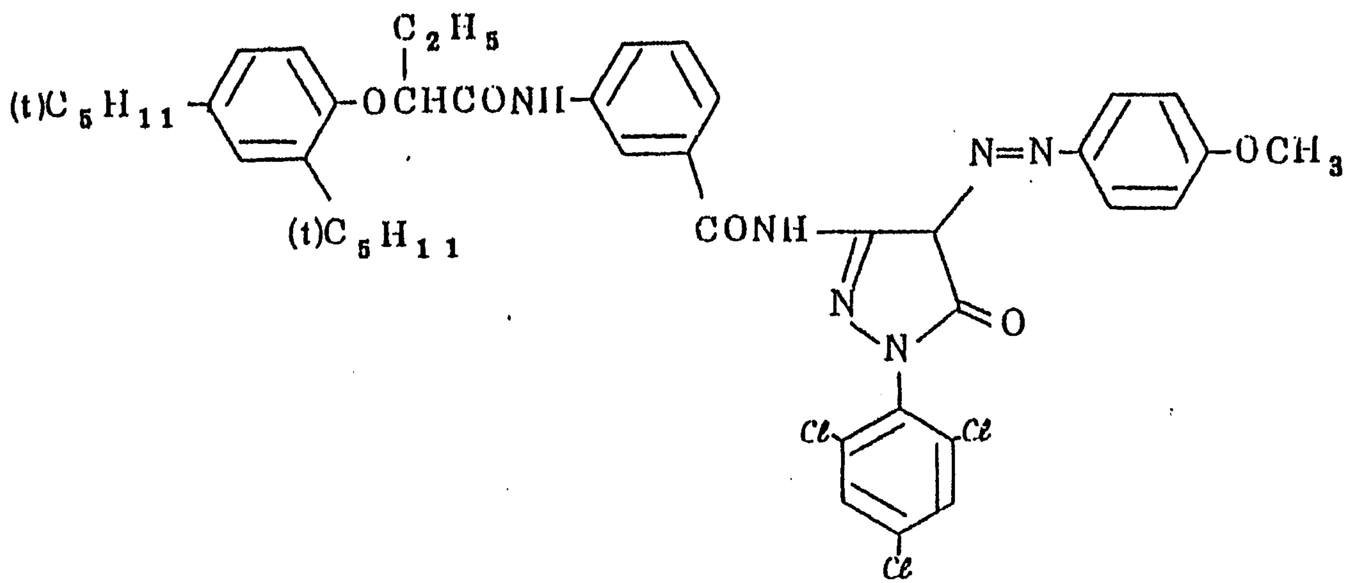

C - 10

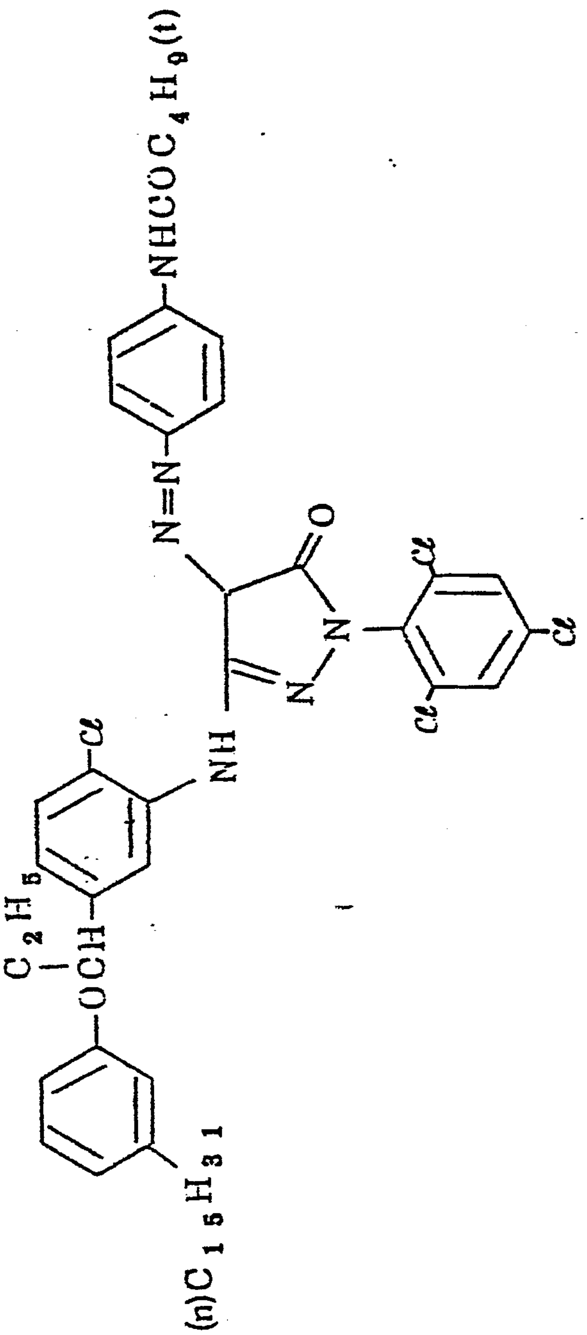

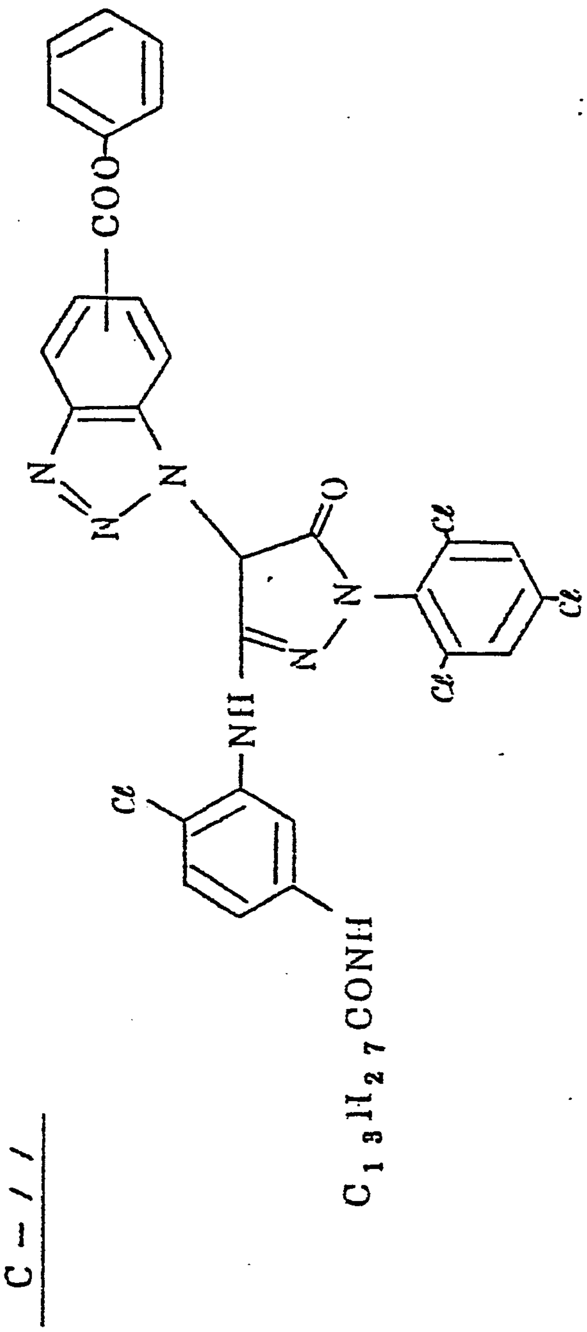

C – 11

C－12

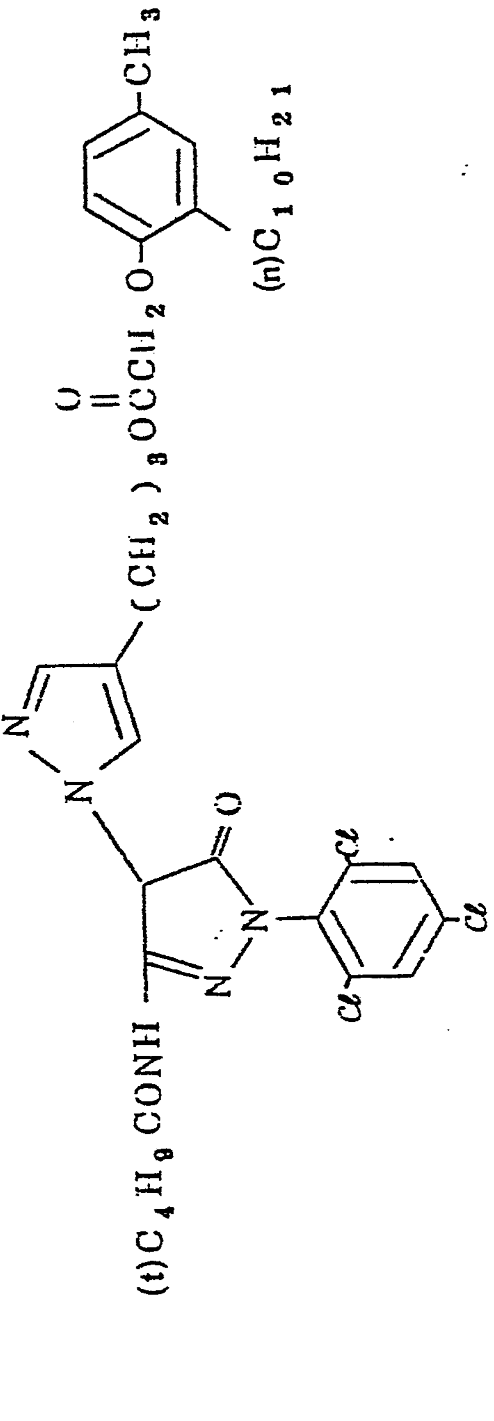

C－13

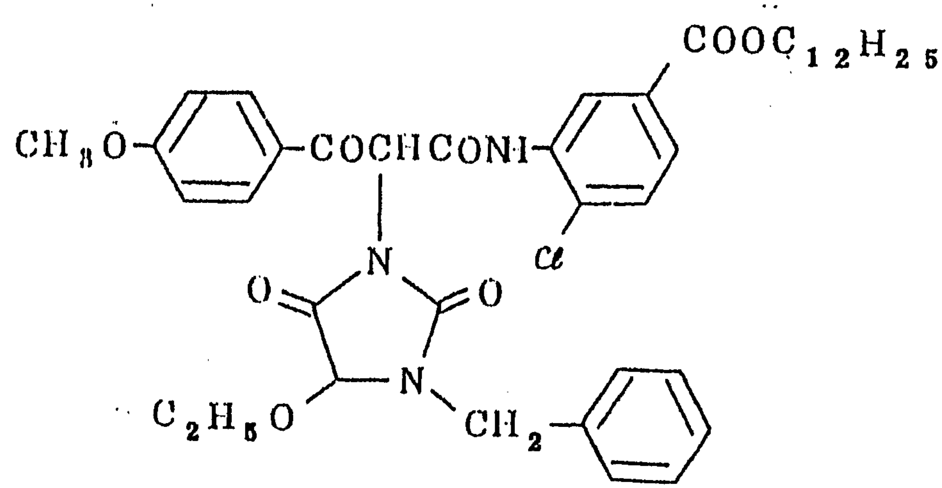

C－14

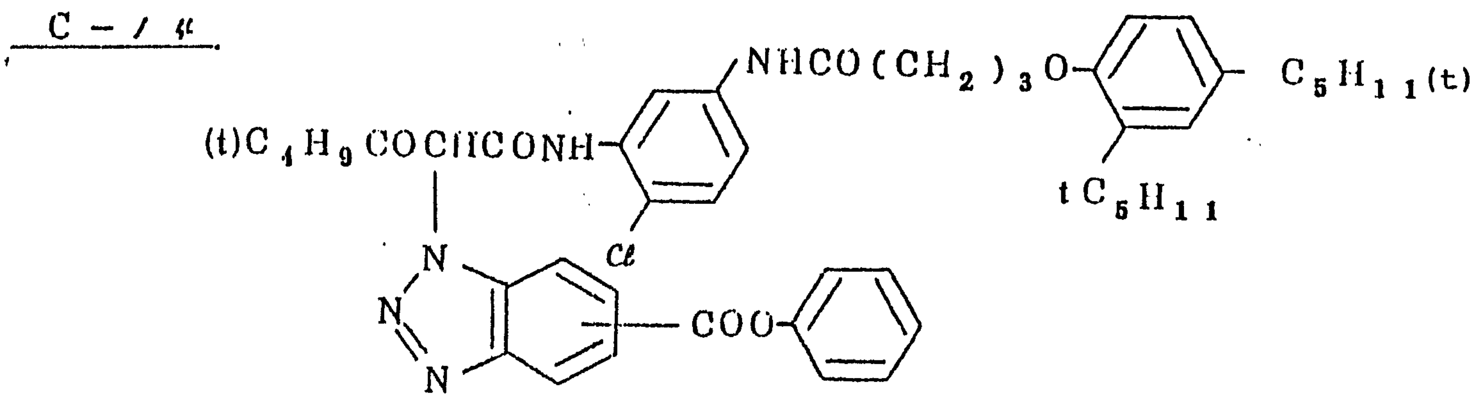

C - 15

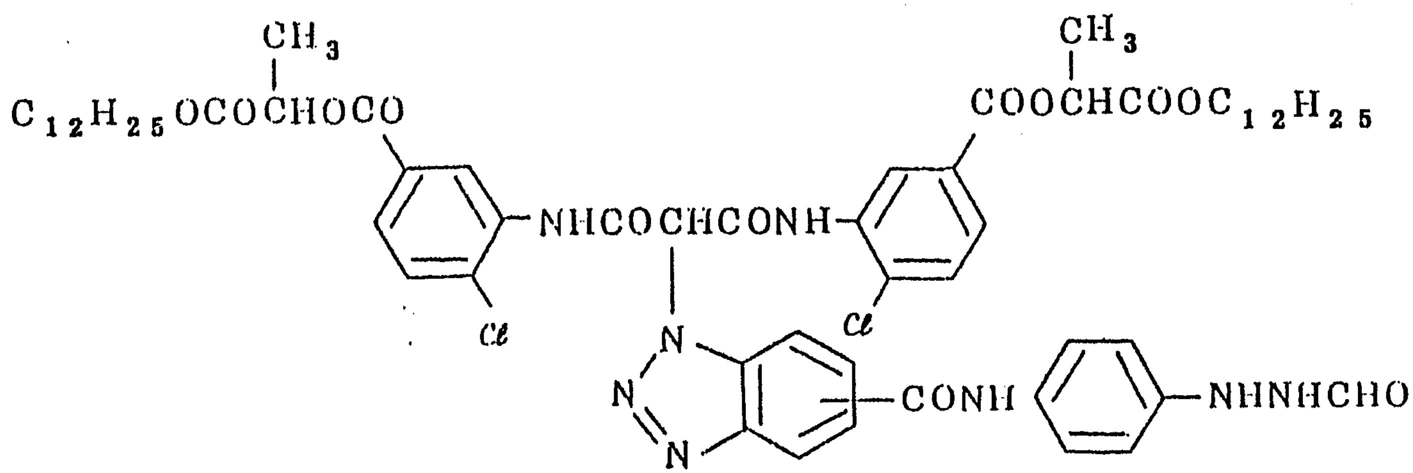

C - 16

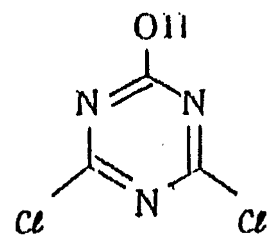

Sensitizing Dyes

I

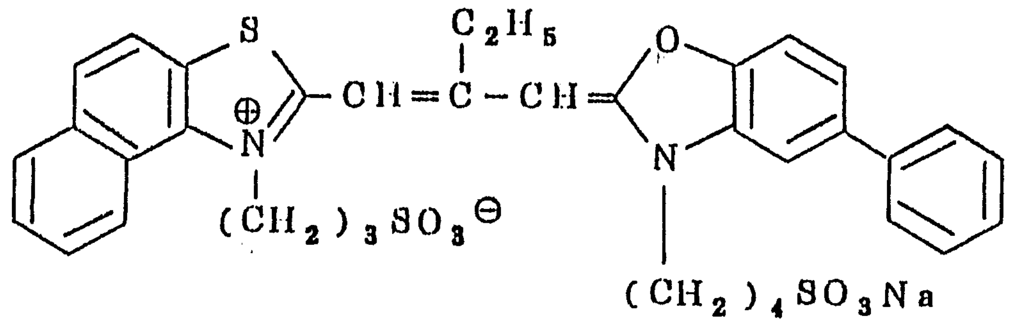

II

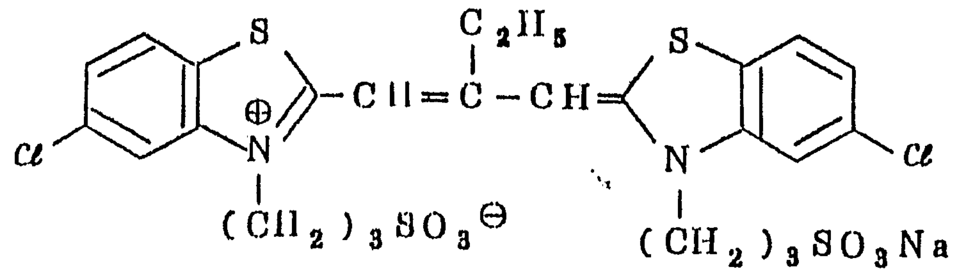

III

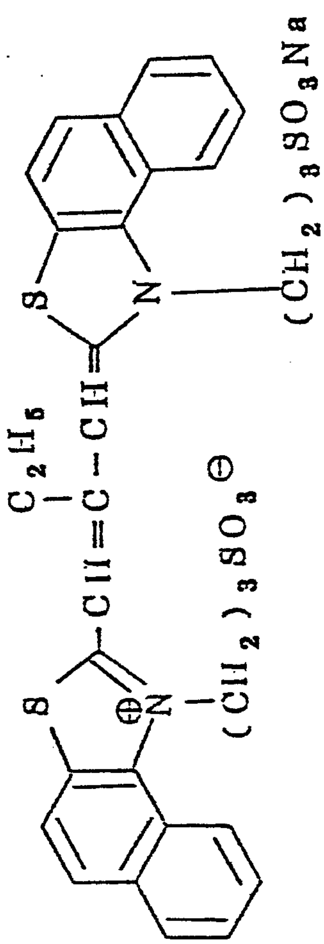

IV

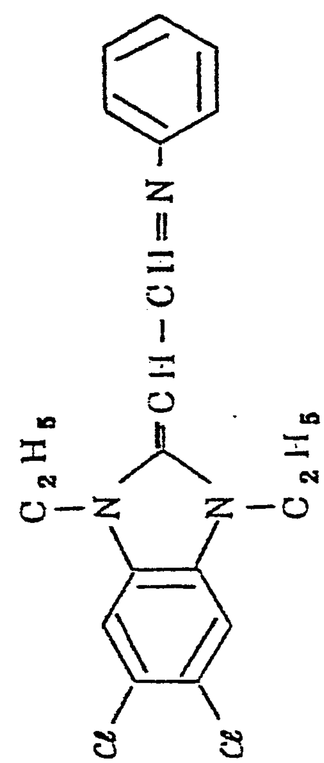

V

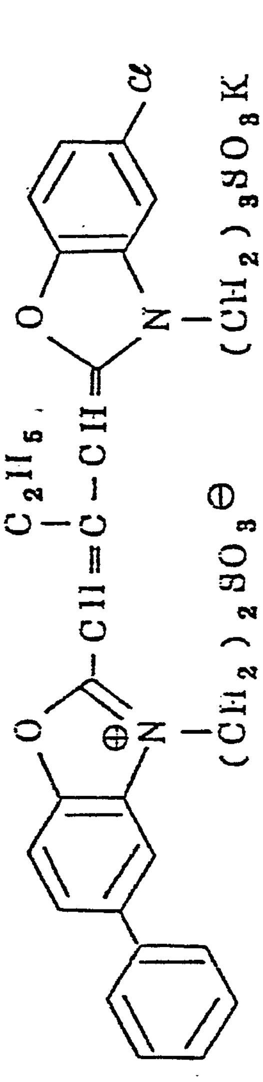

VI

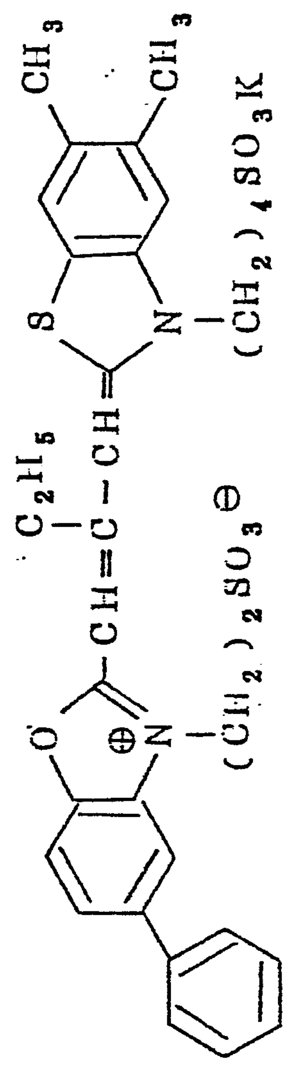

VII

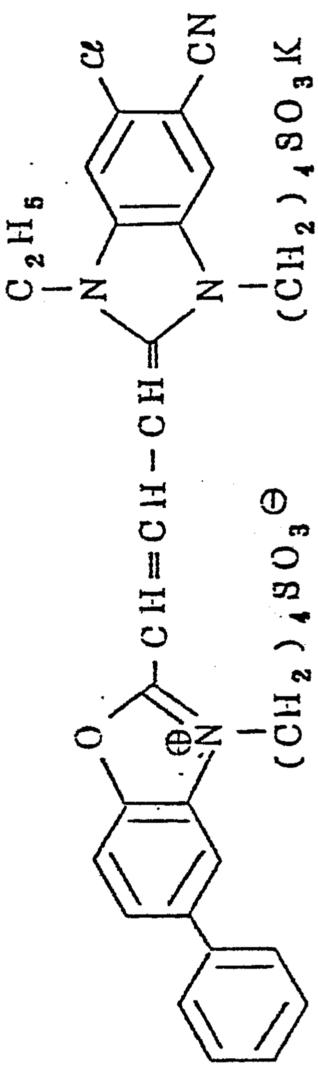

VIII

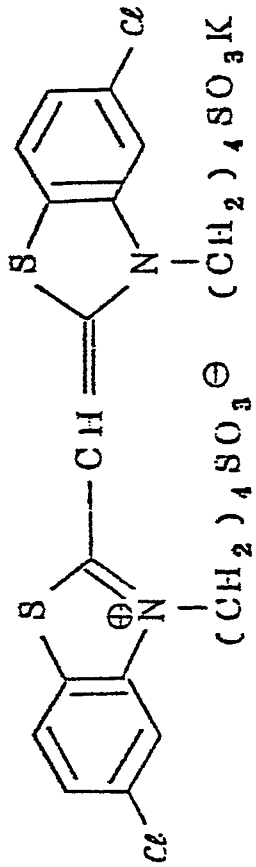

Samples 102 to 106

Samples 102 to 106 were prepared in the same manner as in Sample 1 except that comparison compounds Cp-1, Cp-2 and Cp-3, and Compounds 1 and 16 accoridng the present invention were each used in an amount of 0.2 $mg/m^2$, 0.1 $mg/m^2$, 0.5 $mg/m^2$, 0.5 $mg/m^2$ and 0.1 $mg/m^2$, respectively to the fifth layer (Third Red-Sensitive Emulsion Layer) of Sample 101.

The compounds for comparison used are as follows:

Cp-1

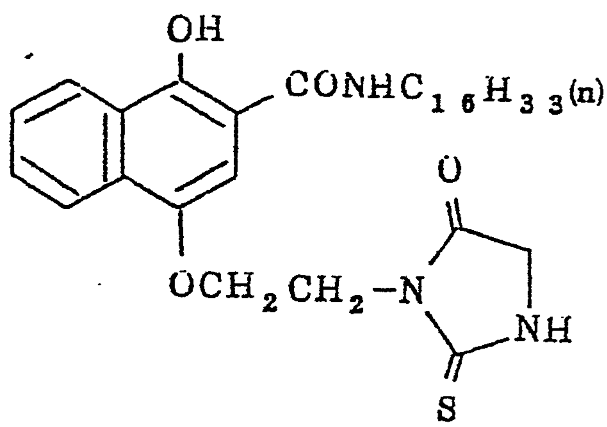

Cp-2

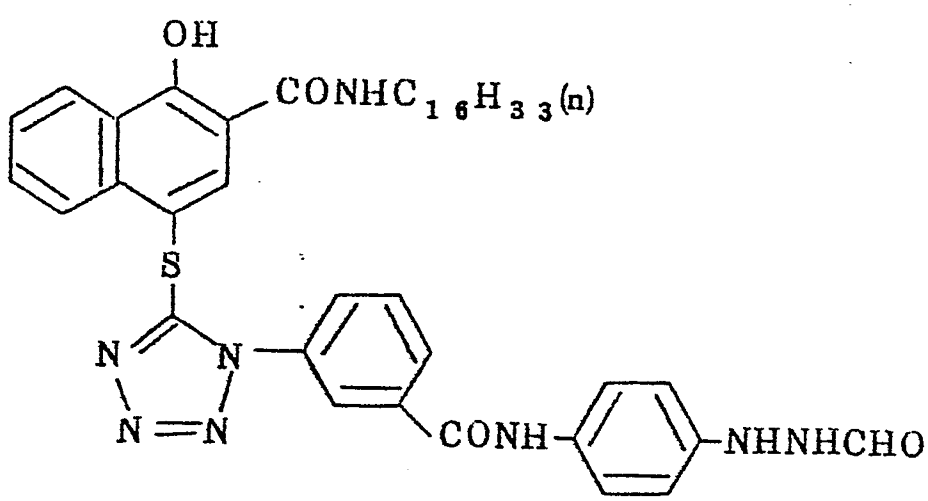

Cp-3

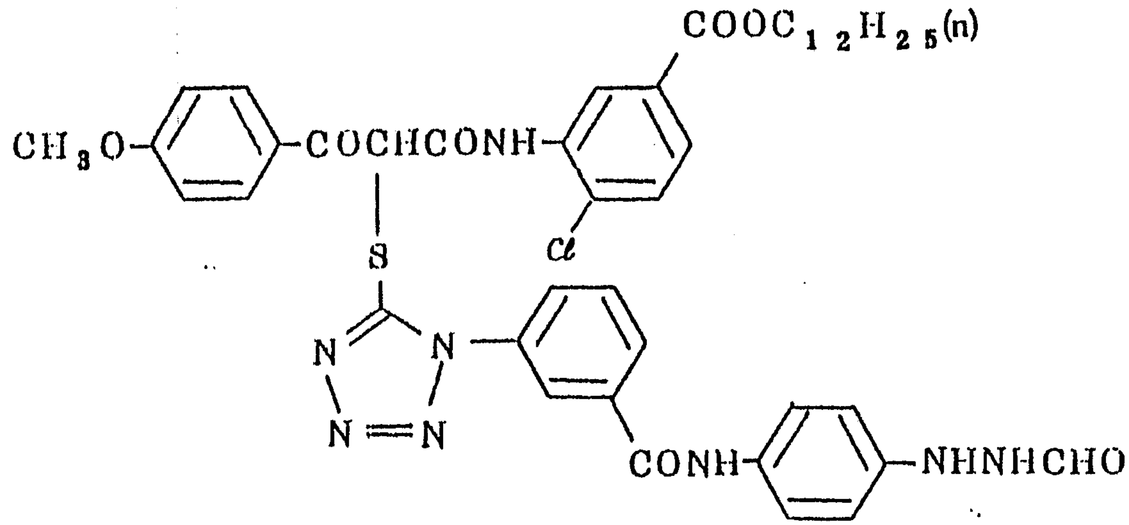

A series of Samples 101 to 106 was kept at room temperature for 14 days (Condition A) and another series of the samples was maintained at 50°C and 80% relative humidity for 14 days (Condition B). These samples were simultaneously subjected to sensitometric exposure and then to the following color development processing at a temperature of 38°C.

| | | |
|---|---|---|
| 1. | Color Development | 2 min. 45 sec. |
| 2. | Bleaching | 6 min. 30 sec. |
| 3. | Washing | 3 min. 15 sec. |
| 4. | Fixing | 6 min. 30 sec. |
| 5. | Washing | 3 min. 15 sec. |
| 6. | Stabilizing | 3 min. 15 sec. |

The processing solutions used above had the following compositions:

Color Developing Solution:

| | |
|---|---|
| Sodium nitrilotriacetate | 1.0 g |
| Sodium sulfite | 4.0 g |
| Sodium carbonate | 30.0 g |
| Potassium bromide | 1.4 g |
| Hydroxylamine sulfate | 2.4 g |
| 4-(N-ethyl-N-β-hydroxyethylamino)-2-methylaniline sulfate | 4.5 g |
| Water to make | 1 liter |

Bleaching Solution:

| | |
|---|---|
| Ammonium bromide | 160.0 g |

| | |
|---|---|
| Aqueous ammonia (28%) | 25.0 ml |
| Sodium iron ethylenediaminetetraacetate | 130 g |
| Glacial acetic acid | 14 ml |
| Water to make | 1 liter |

Fixing Solution:

| | |
|---|---|
| Sodium tetrapolyphosphate | 2.0 g |
| Sodium sulfite | 4.0 g |
| Ammonium thiosulfate (70%) | 175.0 ml |
| Sodium bisulfite | 4.6 g |
| Water to make | 1 liter |

Stabilizing Solution:

| | |
|---|---|
| Formaldehyde | 8.0 ml |
| Water to make | 1 liter |

The density of the thus processed samples was measured using a red filter. The results of photographic properties obtained are shown in Table 1 below.

Table 1

| Sample | Compound Added to Fifth Layer | Condition A | | Condition B | |
|---|---|---|---|---|---|
| | | Fog | Relative Sensitivity 1) | Fog | Relative Sensitivity 1) |
| 101 (Comparison) | - | 0.28 | 100 | 0.27 | 95 |
| 102 (Comparison) | Cp-1 | 0.32 | 132 | 0.35 | 91 |
| 103 (Comparison) | Cp-2 | 0.29 | 126 | 0.31 | 107 |
| 104 (Comparison) | Cp-3 | 0.30 | 129 | 0.28 | 110 |
| 105 (Present Invention) | 1 | 0.29 | 132 | 0.29 | 126 |
| 106 (Present Invention) | 16 | 0.29 | 123 | 0.29 | 120 |

1) Relative Sensitivity: reciprocal of the exposure amount required for obtaining a density of fog + 0.2, when the sensitivity of Sample 101 preserved under Condition A is taken as 100 and the other sensitivities are shown in relation to this value.

From the results shown in Table 1 above, it is apparent that Samples 105 and 106 containing the compounds according to the present invention exhibit only a slight decrease in sensitivity and maintain high sensitivity even when subjected to such severe conditions as Condition B. In contrast, in Comparison Sample 102 to 104 an increase in fog or a remarkable decrease in sensitivity is observed.

EXAMPLE 2

Samples 201 to 204

Samples 201 to 204 were prepared in the same manner as described with Sample 101 except that the comparison compounds Cp-4 and Cp-5, and Compounds 6 and 5 according to the present invention were each used in an amount of 5 $mg/m^2$, 0.5 $mg/m^2$, 0.5 $mg/m^2$ and 5 $mg/m^2$ respectively in place of Coupler C-15 in the fourteenth layer of Sample 101.

The compounds for comparison used are as follows:

Cp-4

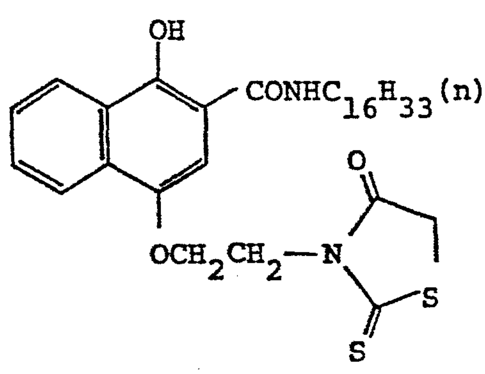

Cp-5

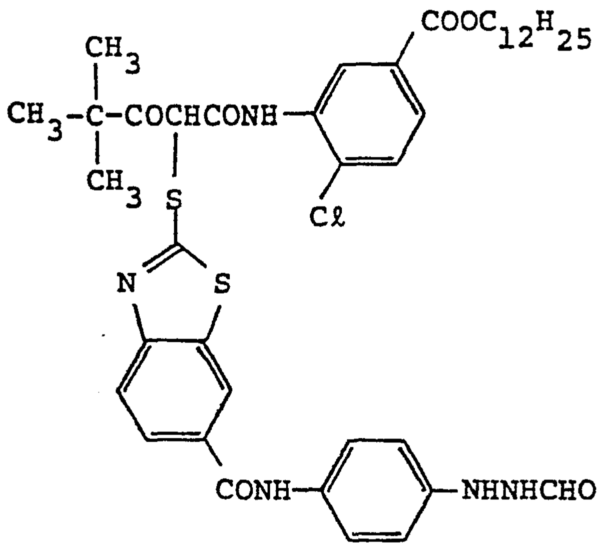

A series of Samples 101 and 201 to 204 was subjected to sensitometric exposure as in Example 1 and kept in a refrigerator for 28 days (Condition C) and another series of the samples was subjected to the same sensitometric exposure and maintained at 40°C and 85% relative humidity for 28 days. These samples were subjected to the same color development processing as described in Example 1. The density of the thus processed samples was measured in the same manner as in Example 1 except using a blue filter. The results obtained are shown in Table 2 below.

Table 2

| Sample | Compound Added to Fourteenth Layer | Condition C | | Condition D | |
|---|---|---|---|---|---|
| | | Minimum Color Density | Relative Sensitivity 1) | Minimum Color Density | Relative Sensitivity 1) |
| 101 (Comparison) | C-15 | 0.90 | 100 | 0.89 | 79 |
| 201 (Comparison) | Cp-4 | 0.93 | 105 | 1.05 | 72 |
| 202 (Comparison) | Cp-5 | 0.90 | 100 | 0.89 | 85 |
| 203 (Present Invention) | 6 | 0.89 | 98 | 0.89 | 95 |
| 204 (Present Invention) | 5 | 0.90 | 103 | 0.90 | 98 |

1) Relative Sensitivity: the sensitivity of Sample 101 (Condition C) is taken as 100 and the other sensitivities in relation to this value.

From the results shown in Table 2 above, it is apparent that Samples 203 and 204 containing the compounds according to the present invention exhibit only a slight decrease in sensitivity under Condition D, indicating the effectiveness of the present invention in preserving stability and high sensitivity even during storage conditions.

While the invention has been described in detail and with reference to specific embodiments thereof, it will be apparent to one skilled in the art that various changes and modifications can be made therein without departing from the spirit and scope thereof.

WHAT IS CLAIMED IS:

1. A silver halide photographic light-sensitive material comprising a support and at least one silver halide emulsion layer wherein the silver halide photographic light-sensitive material contains at least one compound represented by the following general formula (I):

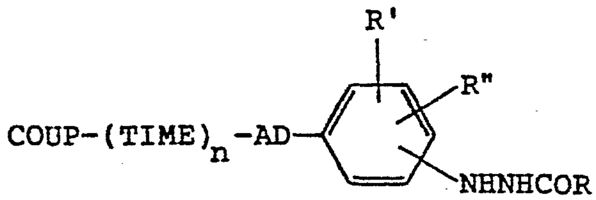

wherein COUP represents a coupler residue capable of undergoing a coupling reaction with an oxidation product of an aromatic primary amine developing agent; TIME represents a timing group which is released from COUP by the coupling reaction and subsequently releases 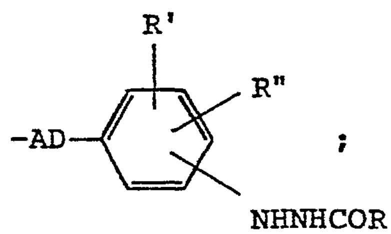 ;

n is 0 or 1; 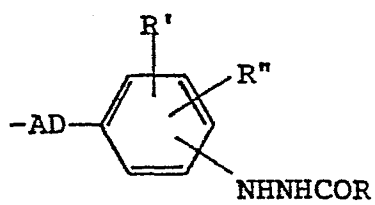 represents a group capable of being released from COUP by the coupling reaction when n is 0, or a group capable of being released from TIME after TIME

has been released from COUP when n is 1, AD being adsorptive to silver halide particles; R represents an alkyl group, an aryl group or a heterocyclic group; R' and R", which may be the same or different, each represents a hydrogen atom or a substituent; and -NHNHCOR may be directly connected to AD when AD is combined with the benzene ring to form a condensed ring system.

2. A silver halide photographic light-sensitive material as claimed in Claim 1, wherein the coupler residue represented by COUP is a residue of a cyan color-forming coupler selected from the group consisting of phenol couplers and naphthol couplers.

3. A silver halide photographic light-sensitive material as claimed in Claim 1, wherein the coupler residue represented by COUP is a residue of a magenta color-forming coupler selected from the group consisting of 5-pyrazolone couplers, pyrazolobenzimidazole couplers, pyrazolotriazole couplers, cyanoacetylcoumarone couplers, open chain acylacetonitrile couplers and indazolone couplers.

4. A silver halide photographic light-sensitive material as claimed in Claim 1, wherein the coupler residue represented by COUP is a residue of a yellow color-forming coupler selected from the group consisting of benzoylacetanilide couplers, pivaloylacetanilide couplers and malondianilide couplers.

5. A silver halide photographic light-sensitive material as claimed in Claim 1, wherein the coupler residue represented by COUP is a residue of a non-color-forming coupler selected from the group consisting of indanones, cyclopentanones, diesters of malonic acid, imidazolinones, oxazolidines and thiazolinones.

6. A silver halide photographic light-sensitive material as claimed in Claim 1, wherein the coupler residue represented by COUP is a residue represented by the following general formula (II), (III), (IV), (V), (VI), (VII), (VIII), (IX) or (X):

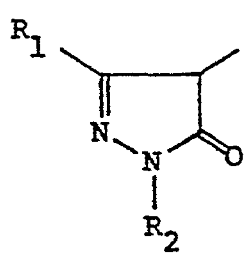

(II)

wherein $R_1$ represents an acylamido group, an anilino group or a ureido group; and $R_2$ represents a phenyl group or a phenyl group substituted with at least one substituent selected from the group consisting of a halogen atom, an alkyl group, an alkoxy group or a cyano group;

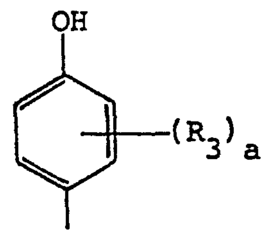

(III)

wherein $R_3$ represents a halogen atom, an acylamido group, or an aliphatic group; a is an integer of 1 to 4, and when a is at least 2, $R_3$ may be the same or different;

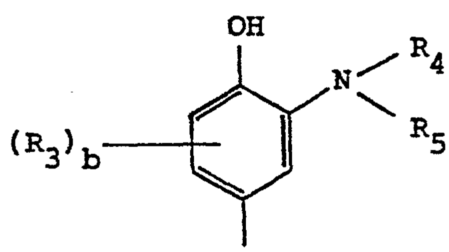

(IV)

wherein $R_3$ has the same meaning as defined in Formula (III) above; $R_4$ and $R_5$, which may be the same or different, each represents an aliphatic group, an aromatic group, a carbamoyl group or a heterocyclic group, provided that either of $R_4$ and $R_5$ may be a hydrogen atom; and b is 0 or an integer of 1 to 3, provided that when b is at least 2, $R_3$ may be the same or different;

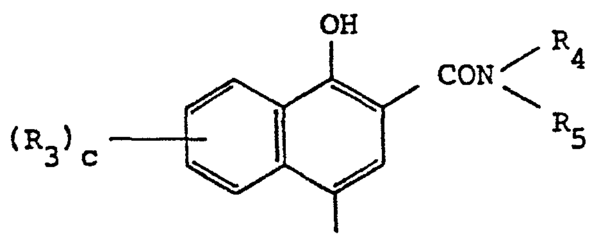

(V)

wherein $R_3$, $R_4$ and $R_5$ each has the same meaning as defined in Formula (IV) above; and c is 0 or an integer of 1 to 5, provided that when c is at least 2, $R_3$ may be the same or different;

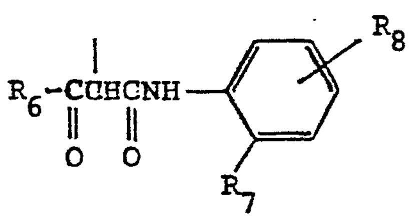

(VI)

wherein $R_6$ represents a tertiary alkyl group or an aromatic group; $R_7$ represents a hydrogen atom, a halogen atom or an alkoxy group; and $R_8$ represents an acylamido group, an aliphatic group, an alkoxycarbonyl group, a sulfamoyl group, a carbamoyl group, an alkoxy group, a halogen atom or a sulfonamido group,

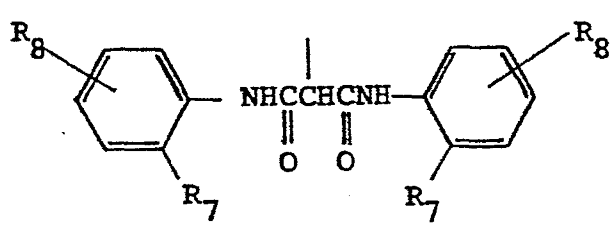

(VII)

wherein $R_7$ and $R_8$ each has the same meaning as defined in Formula (VI) above;

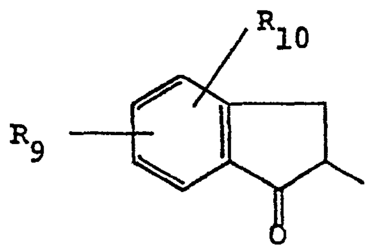

(VIII)

wherein $R_9$ represents an aliphatic group, an alkoxy group, a mercapto group, an alkylthio group, an acylamido group, an alkoxycarbonyl group, a sulfonamido group, a carbamoyl group, a sulfamoyl group, an alkoxysulfonyl group, an aryloxysulfonyl group, an acyl group, a diacylamino group, an alkylsulfonyl group or an arylsulfonyl group; and $R_{10}$ represents a hydrogen atom, a halogen atom, an alkoxy group, an acyl group, a nitro group, an alkylsulfonyl group or an arylsulfonyl group; or the group represented by general formula (VIII) above may be in the form of an enol ester thereof,

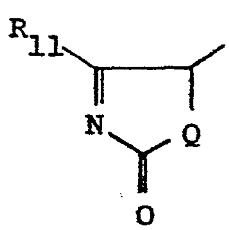

(IX)

wherein $R_{11}$ represents an aliphatic group or an aromatic group; and Q represents an oxygen atom, a sulfur atom or a nitrogen atom,

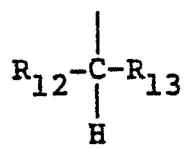 (X)

wherein $R_{12}$ and $R_{13}$, which may be the same or different, each represents a group selected from

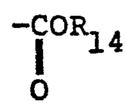 ,

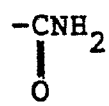 ,

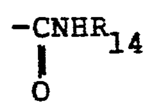 ,

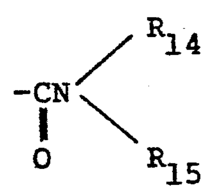 ,

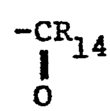 ,

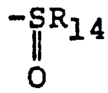 ,

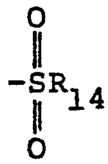 ,

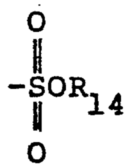 ,

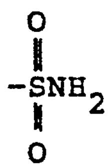 ,

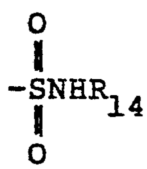 ,

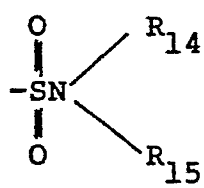 ,

-CN ,

-CHO ,

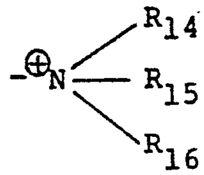
,

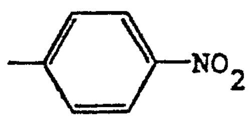

or

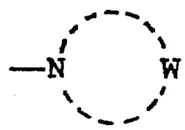

wherein $R_{14}$, $R_{15}$ and $R_{16}$, which may be the same or different, each represents a hydrogen atom, an aliphatic group, an aromatic group or a heterocyclic group; and W represents a non-metallic atomic group necessary to form a 5-memebered or 6-membered ring together with the nitrogen atom; and $R_{12}$ and $R_{13}$ may combine to form a 5-membered or 6-membered ring together with a non-metallic atomic group necessary for completing the ring

7. A silver halide photographic light-sensitive material as claimed in Claim 1, wherein the timing group represented by TIME is selected from (1) a group which is released from COUP by the coupling reaction and subsequently releases the group of 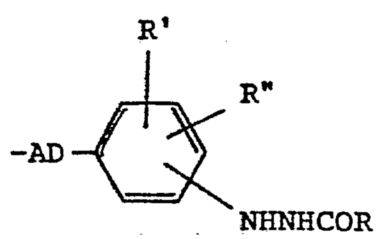 by an intramolecu-

lar displacement reaction, (2) a group which releases the group of 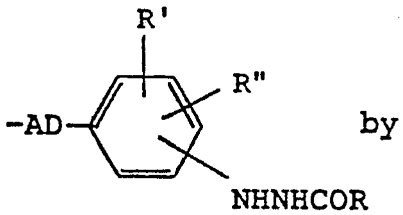 by an electron transfer reaction via a conjugated system, and (3) a group of COUP capable of releasing the group 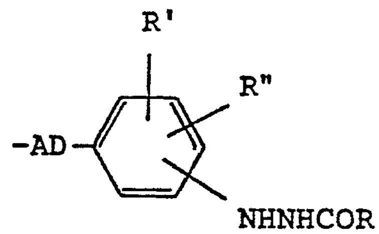 by the coupling reaction with the oxidation product of an aromatic primary amine developing agent.

8. A silver halide photographic light-sensitive material as claimed in Claim 1, wherein the group capable of adsorbing to silver halide grains represented by AD is a group derived from a nitrogen-containing heterocyclic ring having a dissociable hydrogen atom or a group derived from a heterocyclic ring having a mercapto group.

9. A silver halide photographic light-sensitive material as claimed in Claim 8, wherein the nitrogen-containing heterocyclic ring having a dissociable hydrogen atom is selected from pyrrole, imidazole, pyrazole, triazole, tetrazole, benzimidazole, benzopyrazole, benzotriazole, uracil, tetraazaindene, imidazotetrazole, pyrazolotriazole and pentaazaindene.

10. A silver halide photographic light-sensitive material as claimed in Claim 8, wherein the heterocyclic ring having a mercapto group is selected from 2-mercaptobenzothiazole, 2-mercaptopyrimidine, 2-mercaptobenzoxazole and 1-phenyl-5-mercaptotetrazole.

11. A silver halide photographic light-sensitive material as claimed in Claim 1, wherein AD is connected to

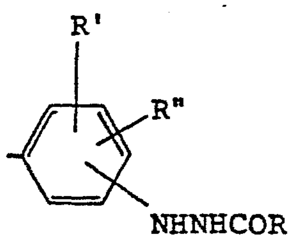

by a divalent linking group selected from an alkylene group, an alkenylene group, an arylene group, -O-, -S-, -CONH-, $-SO_2NH-$, -COO-, -SO-, $-SO_2-$, -NHCONH- and -NHCSNH-.

12. A silver halide photographic light-sensitive material as claimed in Claim 1, wherein R represents an alkyl group having from 1 to 11 carbon atoms, an alkenyl group having from 2 to 11 carbon atoms, an aryl group having from 6 to 12 carbon atoms or a heterocyclic group having from 1 to 12 carbon atoms, and each of these groups may be substituted.

13. A silver halide photographic light-sensitive material as claimed in Claim 1, wherein the substituent represented by R' or R" is an alkyl group, an alkoxy group or a halogen atom.

14. A silver halide photographic light-sensitive material as claimed in Claim 1, wherein the silver halide emulsion layer contains at least one color-forming coupler, and the compound of general formula (I) is present in an amount of from 0.001 to 100% by mole of the total amount of couplers present in the emulsion layer.

15. A silver halide photographic light-sensitive material as claimed in Claim 14, wherein the compound of general formula (I) is present in an amount of from 0.1 to 10% by mole of the total amount of couplers present in the emulsion layer.

16. A silver halide photographic light-sensitive material as claimed in Claim 1, wherein the compound of general formula (I) is present in an amount of from $10^{-8}$ to $10^{-2}$ mole/$m^2$ in a light-insensitive layer.

17. A silver halide photographic light-sensitive material as claimed in Claim 16, wherein the compound of general formula (I) is present in an amount of from $10^{-6}$ to $10^{-3}$ mole/$m^2$ in the light-insensitive layer.

18. A silver halide photographic light-sensitive material as claimed in Claim 1, wherein the silver halide emulsion layer contains the compound of general formula (I).

19. A silver halide photographic light-sensitive material as claimed in Claim 16, wherein the silver halide emulsion layer further contains a color forming coupler.

20. A multilayer color photographic light-sensitive

material comprising a support and at least one blue-sensitive silver halide emulsion layer containing a yellow color forming coupler, at least one green-sensitive silver halide emulsion layer containing a magenta color forming coupler and at least one red-sensitive silver halide emulsion layer containing a cyan color forming coupler wherein at least one of the silver halide emulsion layers contains a compound represented by the following general formula (I):

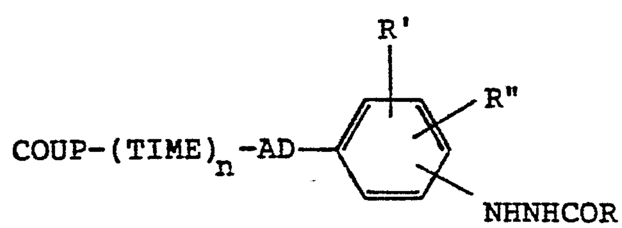

wherein COUP represents a coupler residue capable of undergoing a coupling reaction with an oxidation product of an aromatic primary amine developing agent; TIME represents a timing group which is released from COUP by the coupling reaction and subsequently releases 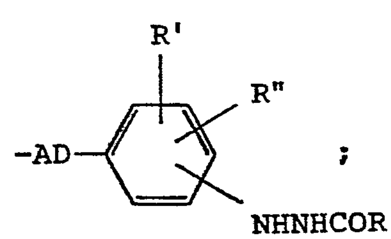 ;

n is 0 or 1; 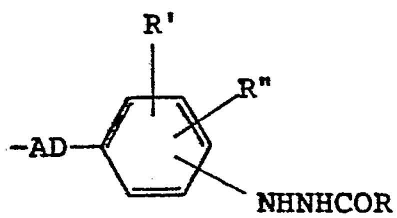 represents a group capable of being released from COUP by the coupling reaction when

n is 0, or a group capable of being released from TIME after TIME has been released from COUP when n is 1, AD being adsorptive to silver halide particles; R represents an alkyl group, an aryl group or a heterocyclic group; R' and R", which may be the same or different, each represents a hydrogen atom or a substituent; and -NHNHCOR may be directly connected to AD when AD is combined with the benzene ring to form a condensed ring system.